(19) Europäisches Patentamt
European Patent Office
Office européen des brevets

(11) Publication number: **0 375 449 B1**

# (12) EUROPEAN PATENT SPECIFICATION

(45) Date of publication of patent specification: **15.02.95**

(51) Int. Cl.[6]: **C07D 311/22**, C07D 311/70, C07D 405/12, C07D 491/04, A61K 31/35, C07D 405/04

(21) Application number: **89313490.8**

(22) Date of filing: **21.12.89**

(54) **Benzopyran-type compounds.**

(30) Priority: **23.12.88 GB 8830222**
**26.05.89 GB 8912168**

(43) Date of publication of application:
**27.06.90 Bulletin 90/26**

(45) Publication of the grant of the patent:
**15.02.95 Bulletin 95/07**

(84) Designated Contracting States:
**AT BE CH DE ES FR GB GR IT LI LU NL SE**

(56) References cited:
**EP-A- 0 214 818**
**AT-B- 330 774**
**US-A- 4 203 895**

(73) Proprietor: **BEECHAM GROUP PLC**
**Beecham House**
**Great West Road**
**Brentford**
**Middlesex TW8 9BD (GB)**

(72) Inventor: **Stemp, Geoffrey Beecham Pharmaceuticals**
**Medicinal Res. Ctr.**
**Coldharbour Road**
**The Pinnacles**
**Fourth Avenue**
**Harlow**
**Essex CM19 5AD (GB)**

(74) Representative: **Tocher, Pauline et al**
**SmithKline Beecham plc**
**Corporate Intellectual Property**
**SB House**
**Great West Road**
**Brentford, Middlesex TW8 9BD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 375 449 B1

## Description

The present invention relates to novel compounds having pharmacological activity, to a process and intermediates for their preparation and their use as pharmaceuticals.

EP-A-76075, 91748, 95316, 120426, 120427, 126367, 126350, 126311, 138134, 139992, 168619, 205292, 214818, 250077 and 321165 (all in the name of Beecham Group p.l.c.) describe classes of compounds which are benzopyran, tetrahydronaphthalene, pyranopyridine or indane derivative potassium channel activators.

EP-A-277611 and 277612 (Hoechst Aktiengesellschaft), EP-A-314446 (American Home Products Corporation) and WO 89/07103 (Nissan Chemical Industries Limited), describe further classes of benzopyran derivatives.

A novel class of compounds has now been discovered, which are also believed to be potassium channel activators, useful in the treatment of disorders associated with smooth muscle contraction. Such disorders include hypertension, including pulmonary hypertension, and cardiovascular disorders other than hypertension, such as congestive heart failure, angina, peripheral vascular disease and cerebral vascular disease. Other disorders include those of the gastrointestinal tract, respiratory system, uterus and urinary tract. Such disorders include irritable bowel syndrome and diverticular disease; reversible airways obstruction and asthma; premature labour; incontinence and kidney stones. They may also be of potential use in the treatment of epilepsy.

Accordingly, the present invention provides a compound of formula (I), or a pharmaceutically acceptable salt thereof:

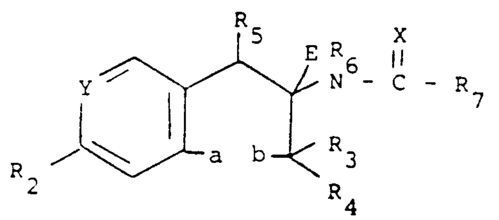

(I)

wherein

a and b together form an -O- linkage or a bond or (when $R_2$ is hydrogen), $CH_2$;

either Y is N and $R_2$ is hydrogen; or

Y is C-$R_1$

wherein

either one of $R_1$ and $R_2$ is hydrogen and the other is nitro, cyano, halo, $CF_3$, $C_2F_5$, formyl, aldoxime, $CF_3O$, $NO_2$-CH=CH-, NC-CH=CH-; a group $R_xX$-wherein $R_x$ is $C_{1-6}$ alkyl, aryl or heteroaryl either of which may be optionally substituted by one, two or three of $C_{1-4}$ alkyl, $C_{1-4}$ alkoxy, nitro, halo, $CF_3$ and cyano; and X is C=O, O.C=O, C=O.O, CHOH, SO, $SO_2$, O.SO, O.$SO_2$, CONH, O.CONH, O.$SO_2$NH, CO-CH=CH, C=NHOH, C=$NNH_2$; or a group $R_yR_zNZ$- wherein $R_y$ and $R_z$ are independently hydrogen or $C_{1-6}$ alkyl and Z is C=O, SO or $SO_2$; or a group $(R_wO)_2P(O)W$ wherein $R_w$ is hydrogen or $C_{1-6}$ alkyl and W is O or a bond; or

$R_1$ is a $C_{3-8}$ cycloalkyl group or a $C_{1-6}$ alkyl group optionally substituted by a group which is hydroxy, $C_{1-6}$ alkoxy, amino optionally substituted by one or two $C_{1-6}$ alkyl groups, $C_{1-7}$ alkanoylamino, $C_{3-8}$ cycloalkyloxy or $C_{3-8}$ cycloalkylamino; and $R_2$ is hydrogen; or

one of $R_1$ and $R_2$ is nitro, cyano or $C_{1-3}$ alkylcarbonyl and the other is a different group selected from nitro, cyano, halo, $C_{1-3}$ alkylcarbonyl, methoxy or amino optionally substituted by one or two $C_{1-6}$ alkyl or by $C_{2-7}$ alkanoyl; or

$R_1$ and $R_2$ together with the carbon atoms to which they are attached, form 2,1,3-oxadiazole;

either one of $R_3$ and $R_4$ is hydrogen or $C_{1-4}$ alkyl and the other is $C_{1-4}$ alkyl; or $R_3$ and $R_4$ together are $C_{2-5}$ polymethylene.

$R_5$ is hydrogen, hydroxy, amino, $C_{1-6}$ alkoxy or $C_{1-7}$ acyloxy; and

E is hydrogen; or

$R_5$ and E together form a bond;

either $R_6$ is hydrogen or $C_{1-6}$ alkyl; and

$R_7$ is hydrogen, $C_{3-8}$ cycloalkyl, $C_{2-6}$ alkenyl, $C_{1-6}$ alkyl optionally substituted by hydroxy, $C_{1-6}$ alkoxy, $C_{1-6}$ alkoxycarbonyl, carboxy, halogen or amino optionally substituted by one or two $C_{1-6}$ alkyl groups; aryl or heteroaryl either being optionally substituted by one or more groups or atoms selected from the class of $C_{1-6}$ alkyl, $C_{1-6}$ alkoxy, hydroxy, halogen, trifluoromethyl, nitro, cyano, $C_{1-12}$ carboxylic acyl, or $R_7$ is amino or aminocarbonyl optionally substituted by one or two $C_{1-6}$ alkyl groups or substituted by $C_{3-8}$ cycloalkyl, or a group $OR_9$ wherein $R_9$ is $C_{1-6}$ alkyl or benzyl optionally substituted as defined for $R_7$; or

$R_6$ and $R_7$ are joined together to form $C_{3-4}$ polymethylene optionally substituted by one or two $C_{1-6}$ alkyl groups and optionally containing one or, (when $C_4$ polymethylene), two, C=C double bond(s); and

X is oxygen or sulphur.

a and b are preferably an -O- linkage and Y is preferably C-$R_1$ as defined.

When either one of $R_1$ and $R_2$ is hydrogen, the other is preferably selected from halo, $CF_3$, $C_2F_5$, $C_{1-6}$ alkylcarbonyl, $C_{1-6}$ alkoxycarbonyl, nitro or cyano.

When one of $R_1$ and $R_2$ is nitro, cyano or $C_{1-3}$ alkylcarbonyl the other is, favourably, amino optionally substituted by one or two $C_{1-6}$ alkyl groups or by $C_{2-7}$ alkanoyl. In particular, when one of $R_1$ and $R_2$ is nitro, cyano or acetyl, the other is amino, methylamino, dimethylamino or acetylamino. Preferably, when one or $R_1$ and $R_2$ is nitro or cyano, especially cyano, the other is amino.

Halo substituents in $R_1$ and/or $R_2$ are usually chloro or bromo.

Values for $R_x$ when alkyl in $R_1/R_2$ are usually selected from methyl, ethyl, n- and iso-propyl, n-, iso-, sec-and tert-butyl, preferably methyl or ethyl. Suitable examples of other alkyl or alkyl containing groups in $R_1$ and in $R_3$ to $R_7$ when alkyl include those listed for $R_1$ and $R_2$ alkyl groups. Cycloalkyl groups include $C_3$, $C_4$, $C_5$, $C_6$, $C_7$ and $C_8$ cycloalkyl.

When $R_2$ is hydrogen, a preferred value for $R_1$ is $C_{1-6}$ alkyl, such as methyl, ethyl, iso-propyl or t-butyl.

A sub-group of $R_x$ or $R_7$ heteroaryl is 5- or 6-membered monocyclic or 9- or 10-membered bicyclic heteroaryl of which 5- or 6-membered monocyclic heteroaryl is preferred. In addition, 5- or 6-membered monocyclic or 9- or 10-membered bicyclic heteroaryl preferably contains one, two or three heteroatoms which are selected from the class of oxygen, nitrogen and sulphur and which, in the case of there being more than one heteroatom, are the same or different. Examples of 5- or 6-membered monocyclic heteroaryl containing one, two or three heteroatoms which are selected from the class of oxygen, nitrogen and sulphur include furyl, thienyl, pyrryl, oxazolyl, thiazolyl, imidazolyl and thiadiazolyl, and pyridyl, pyridazyl, pyrimidyl, pyrazyl and triazyl. Preferred examples of such groups include furyl, thienyl, pyrryl and pyridyl, in particular 2- and 3-furyl, 2- and 3-pyrryl, 2- and 3-thienyl, and 2-, 3- and 4-pyridyl. Examples of 9- or 10-membered bicyclic heteroaryl containing one, two or three heteroatoms which are selected from the class of oxygen, nitrogen and sulphur include benzofuranyl, benzothienyl, indolyl and indazolyl, quinolyl and isoquinolyl, and quinazonyl. Preferred examples of such groups include 2- and 3-benzofuryl, 2- and 3-benzothienyl, and 2- and 3-indolyl, and 2- and 3-quinolyl.

$R_x$ or $R_7$ when aryl is usually phenyl or naphthyl.

Examples of the groups or atoms for optional substitution of $R_x$ or $R_6$ when aryl or heteroaryl include methyl, methoxy, hydroxy, fluoro, chloro, nitro or cyano.

$R_1$ is preferably nitro, cyano, acetyl, $CF_3$, $C_2F_5$ or $C_{1-4}$ alkyl.

Preferably $R_3$ and $R_4$ are both methyl groups.

Suitable examples of $R_5$ when alkoxy include methoxy, ethoxy, n- and iso-propoxy, of which methoxy is preferred. When $R_5$ is $C_{1-7}$ acyloxy it is usually $C_{1-7}$ carboxylic acyloxy, such as $C_{1-7}$ alkanoyloxy wherein the alkyl moiety is usually as listed for alkyl in $R_1$ and $R_2$ above.

$R_5$ is preferably hydroxy.

Preferably $R_6$ and $R_7$ are not joined, and $R_6$ is hydrogen and $R_7$ is optionally substituted phenyl, preferably 4-fluorophenyl, or branched $C_{1-6}$ alkyl, such as t-butyl; $OR_9$ wherein $R_9$ is $C_{1-6}$ alkyl or benzyl, or $R_7$ is optionally substituted amino as defined, most preferably t-butylamino.

Examples of pharmaceutically acceptable salts include acid addition salts with acids such as hydrochloric, hydrobromic, phosphoric, sulphuric, citric, tartaric, lactic or acetic acid.

The compounds of formula (I) have at least one asymmetric centre and therefore exist in more than stereoisomeric form. The invention extends to each of these forms individually and to mixtures thereof, such as racemates.

The $R_6NCXR_7$ moiety may be cis or trans to the $R_5$ group when $R_5$ is hydroxy, amino, $C_{1-6}$ alkoxy or $C_{1-7}$ acyloxy. The trans configuration is preferred.

The compounds of formula (I) and their salts may form solvates, such as hydrates, and these are included as an aspect of the invention, wherever a compound of formula (I) or a salt thereof is herein

referred to.

The invention also provides a process for the preparation of a compound of formula (I), or a pharmaceutically acceptable salt thereof, which process comprises acylating a compound of formula (II):

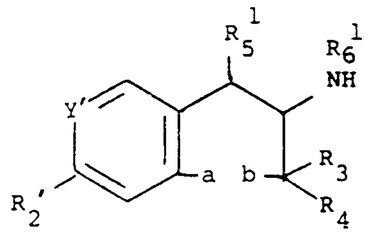

(II)

wherein Y′ and $R_2'$ are Y and $R_2$ respectively or groups or atoms convertible thereto, $R_5^1$ is hydroxy, $C_{1-6}$ alkoxy, $C_{1-7}$ acyloxy or azide, $R_6^1$ is hydrogen or $C_{1-6}$ alkyl and the $R_6^1$NH group is trans to the $R_5^1$ group (when $R_5^1$ is other than than azide), with

a) an acylating agent of formula (III):

$R_7^1$COQ (III)

wherein Q is a leaving group and $R_7^1$ is $R_7$ as defined or a group convertible thereto, when not joined to $R_6$ or, (when $R_6^1$ is hydrogen), $R_7^1$ is a group $R_{10}$ which is $C_{3-4}$ polymethylene optionally substituted by one or two $C_{1-6}$ alkyl groups and optionally containing one or, (when $C_4$ polymethylene), two, C=C double bond(s), terminally substituted by a leaving group, L; and thereafter, when $R_7^1$ is $R_{10}$, cyclising the resulting compound; or

b) (when $R_7$ is $NHR_{11}$ wherein $R_{11}$ is hydrogen or $C_{1-6}$ alkyl), with a compound of formula (IV):

X = C = $NR_{11}$ (IV)

wherein X and $R_{11}$ are as hereinbefore defined;

and thereafter, as desired or necessary, converting $R_5^1$ to $R_5$, converting Y′ and/or $R_2'$ to Y and/or $R_2$, optionally thiating X when oxygen to X is sulphur and/or dehydrating a compound wherein $R_5^1$ is hydroxy to form a compound of formula (I) wherein $R_5$ and E form a bond or converting a compound of formula (I) wherein the $R_6NCXR_7$ moiety is trans to $R_5$ when hydroxy, to a corresponding cis isomer; and optionally forming a pharmaceutically acceptable salt thereof.

In the process variant a) acylation of a compound of formula (II) with an acylating agent of formula (III), the leaving group Q is a group that is displaceable by a primary or secondary amino nucleophile. Examples of such a group include $C_{1-4}$ alkanoyloxy, and halogen, such as chloro and bromo. When the leaving group Q is either of these examples, the acylating agent of formula (III) is either an acid anhydride or an acid halide. When it is an acid anhydride, it may be a mixed or simple anhydride. If it is a mixed anhydride, it may be prepared in situ from a carboxylic acid and an acid halide, although this is less preferred than using the halide itself.

In process variant a), when $R_7$ in the desired compound of formula (I) is an $R_7$ optionally substituted amino-substituted alkyl group as hereinbefore defined, it is preferred that $R_7^1$ is a group convertible to the $R_7$ substituted alkyl group as hereinbefore defined, in particular that it is $C_{1-6}$ alkyl substituted by halo, especially bromo. The $R_7^1$ halo substituent in the resultant compound of process variant a) may be converted to an $R_7$ substituent which is amino optionally substituted as hereinbefore defined, by a conventional amination reaction with ammonia or a corresponding alkyl- or dialkylamine.

Less favourably $R_7^1$ may be $C_{1-6}$ alkyl substituted by protected amino, protected $C_{1-6}$ alkylamino or amino substituted by two independent $C_{1-6}$ alkyl groups, it being necessary to protect the $R_7$ amino function in process variant a).

When the acylating agent of formula (III) is an acid anhydride, the acylation of the compound of formula (II) may be carried out in the presence of an acid acceptor, such as sodium acetate, optionally using the anhydride as the solvent.

When the acylating agent of formula (III) is an acid halide, the acylation of the compound of formula (II) is, preferably, carried out in a non-aqueous medium, such as dichloromethane, in the presence of an acid

acceptor, such as triethylamine, trimethylamine, pyridine, picoline or calcium, potassium or sodium carbonate.

When $R_5$ in a compound of formula (II) is hydroxy, there is a risk of a side-reaction between the hydroxy group and the acylating agent of formula (III). However, the reaction may be carried out under controlled conditions such that only the $R_6{}^1$NH- is acylated, for example, by using a $C_{2-9}$ acyloxy group as the leaving group Q, in the acylating agent of formula (III) in the manner as previously described for an acid anhydride, and/or effecting the reaction at relatively low temperature, e.g. at below 10°C. Alternatively $R_5$ may be $C_{1-7}$ acyloxy in a compound of formula (II), although less preferably if $R_5$ in the resultant compound of formula (I) is to be hydroxy, and, after reaction with the acylating agent of formula (III), be converted into hydroxy, as described hereinafter.

When $R_7{}^1$ is $R_{10}$ as hereinbefore defined, the leaving group L is a group that is displaceable by a secondary amino nucleophile adjacent to a carbonyl function. A preferred example is chloro.

The cyclisation reaction when $R_7{}^1$ is $R_{10}$ where the variables are as hereinbefore defined is preferably carried out in an inert solvent such as tetrahydrofuran or dimethylformamide.

In process variant b), the reaction is preferably carried out in an inert solvent, such as dichloromethane, at temperatures at ambient or below, such as below 10°C. When $R_{11}$ is hydrogen in formula (IV), the reaction is preferably carried out using a corresponding alkali metal cyanate or thiocyanate, for example, that of sodium or potassium in an optionally methanolic aqueous medium acidified with a mineral acid, such as dilute hydrochloric acid, at an elevated temperature 50 to 90°C.

Examples of a conversion of $R_5{}^1$ into $R_5$ are generally known in the art. For example, when $R_5{}^1$ is hydroxy, it may be alkylated using an alkyl iodide in an inert solvent, such as toluene, in the presence of a base, such as potassium hydroxide, or it may be acylated using a carboxylic acid chloride or anhydride in a non-hydroxylic solvent in the presence of an acid acceptor. Alternatively, when $R_5{}^1$ is $C_{1-7}$ acyloxy or $C_{1-6}$ alkoxy, it may be converted into hydroxy by conventional hydrolysis with, for example, dilute mineral acid.

When $R_5{}^1$ is azide, it may be converted to $R_5$ is amino by reduction, preferably using propane-1,3-dithiol, as described in Example 26 hereinafter.

Suitable conversions of Y′/$R_2$′ are as described in aforementioned European Patent Publications.

The optional thiation of the $R_6$-N-CO-$R_7$ group in a compound of formula (I) to give another compound of formula I, wherein X is sulphur, is, preferably, carried out with conventional thiation agents, such as hydrogen sulphide, phosporous pentasulphide and Lawesson's reagent (p-methoxyphenylthiophosphine sulphide dimer). The use of hydrogen sulphide and phosporous pentasulphide may lead to side-reactions and, therefore, the use of Lawesson's reagent is preferred. The thiation reaction conditions are conventional for the thiation agent employed. For example, the use of hydrogen sulphide is, preferably, acid catalysed by, for example, hydrogen chloride in a polar solvent, such as acetic acid or ethanol. The preferred use of Lawesson's reagent is, preferably, carried out under reflux in a dry solvent, such as toluene or dichloromethane.

The dehydration of a resulting compound of formula (I) wherein $R_5$ is hydroxy and E is hydrogen may be carried out under conventional dehydration conditions, using a dehydrating agent such as sodium hydride, in an inert solvent, such as dry tetrahydrofuran, at reflux temperature.

Conversion of a compound of formula (I) wherein the $R_6NCXR_7$ moiety is trans to $R_5$ when hydroxy, to a corresponding cis isomer, may be achieved according to the method described by C.S.V. Houge Frydrych and I.L. Pinto, Tetrahedron Letters 30, 1989, p3349.

The optional formation of a pharmaceutically acceptable salt, when the resulting compound of formula (I) contains a salifiable group, may be carried out conventionally.

The intermediates of formula (II) wherein $R_6{}^1$ is hydrogen and $R_5$ is OH, are prepared by hydrolysis of an aziridine intermediate of formula (V):

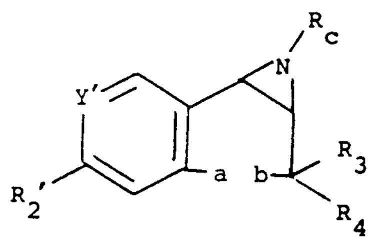

(V)

wherein $R_c$ is an amino protecting group and the remaining variables are as hereinbefore defined; followed by deprotection, if necessary.

Examples of the group $R_c$ include t-butyloxycarbonyl, ethoxycarbonyl, methoxycarbonyl and benzyloxycarbonyl, removed by conventional methods. The t-butyloxycarbonyl group is preferred, in which case, the deprotection preferably takes place under acidic conditions, using trifluoroacetic acid, in an inert solvent, such as dichloromethane at a temperature of around 0°C.

The trans intermediates of formula (II) wherein $R_6{}^1$ is hydrogen and $R_5$ is azide, are prepared by reacting an intermediate of formula (V) as hereinbefore defined, with sodium azide, as described in Description 15 hereinafter.

The cis intermediates of formula (II) wherein $R_6{}^1$ is hydrogen and $R_5$ is azide may be prepared from the intermediates of formula (VI), as hereinafter defined, by reaction with sodium azide.

When $R_6{}^1$ is other than hydrogen, the intermediates of formula (II) are prepared from the corresponding intermediate wherein $R_6{}^1$ is hydrogen, by conventional methods of forming secondary amines, i.e. by alkylation or reductive amination.

Compounds of the formula (V) are prepared by reacting a compound of formula (VI):

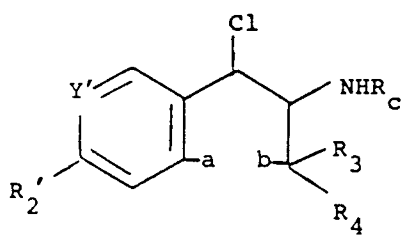

(VI)

wherein the chloro substituent is trans to the $NHR_c$ substituent, with a base, such as potassium carbonate, as described in Description 2 hereinafter; or (when a and b together are a bond), with sodium hydride/DMF/40°C.

Compounds of the formula (VI) are prepared by reacting a compound of formula (VII):

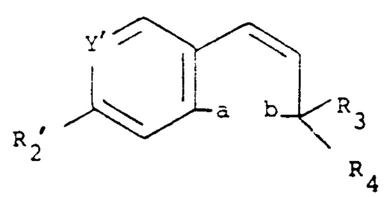

(VII)

with, when $R_c$ is t-butyloxycarbonyl, t-butyl-N,N-dichlorocarbamate, in a similar manner to that described in Description 1 hereinafter. Another suitable solvent is carbon tetrachloride and it will be appreciated that the choice of solvent will be influenced by the value of Y' and/or a and b.

Compounds of the formula (VII) are described in the aforementioned Patent Publications, prepared by the process depicted below (when a and b together form an -O- linkage):

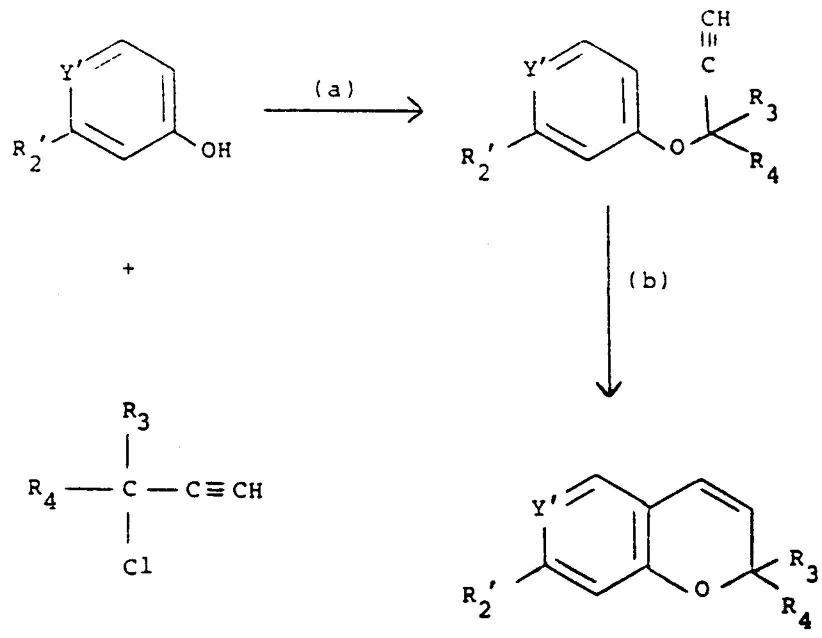


(VII)

(a) Reflux; acetone; $K_2CO_3$/KI; or a phase transfer catalytic method with optional sonification;
(b) Heat in 1,2-dichlorobenzene or N,N-diethylaniline;

When $R_c$ is t-butyloxycarbonyl, the intermediates of formula (II) wherein $R_6{}^1$ is hydrogen and $R_5$ is hydrogen are prepared from the intermediates of formula (VII) by reaction with t-butyl-N,N-dichlorocarbamate, followed by ammonium acetate/zinc dust in ethanolic dioxan, as described in Example 4 hereinafter.

It will be appreciated that, when $R_6$ is hydrogen and $R_7$ in the desired compound of formula (I) is $C_{1-6}$ alkoxycarbonyl, $R_c$ may be identical to $R_7$ and therefore deprotection and subsequent acylation of the compound of formula (II) is not necessary (see Examples 1 and 4 hereinafter).

As mentioned previously, some of the compounds of formula (I) may exist in optically active forms, in particular it will be appreciated that the carbon atoms to which E and $R_5$ are attached are asymmetric when $R_5$ and E together do not form a bond. The individual enantiomers may be resolved by conventional methods; or they may be prepared from resolved intermediates of formula (II).

It is preferred that the compounds of formula (I) are isolated in substantially pure pharmaceutically acceptable form.

The intermediates of formulae (II), (V) and (VI) are novel and represent an aspect of the present invention.

As mentioned previously, the compounds of formula (I) have been found to have blood-pressure lowering activity and/or bronchodilator activity. They are therefore useful in the treatment of hypertension. They are also believed to be of potential use in the treatment of other disorders hereinbefore referred to.

The present invention accordingly provides a pharmaceutical composition which comprises a compound of formula (I) or a pharmaceutically acceptable salt thereof, and a pharmaceutically acceptable carrier. In particular, the present invention provides an anti-hypertensive or bronchodilator pharmaceutical composition which comprises an effective amount of a compound of formula (I) or a pharmaceutically acceptable salt thereof, and a pharmaceutically acceptable carrier.

The compositions are preferably adapted for oral administration. However, they may be adapted for other modes of administration, for example parenteral administration for patients suffering from heart failure. Other alternative modes of administration include sublingual or transdermal administration. A composition may be in the form of spray, aerosol or other conventional method of inhalation, for treating respiratory tract disorders.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel,hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight, of the active material, depending on the method of administration.

The present invention further provides a method of prophylaxis or treatment of hypertension or respiratory tract disorders in mammals including man, which comprises administering to the suffering mammal an anti-hypertensive or bronchodilator effective amount of a compound of formula (I) or a pharmaceutically acceptable salt thereof.

An effective amount will depend on the relative efficacy of the compounds of the present invention, the severity of the disorder being treated and the weight of the sufferer. However, a unit dose form of a composition of the invention may contain from 0.05 to 500 mg of a compound of the invention and more usually from 0.1 to 50 mg, for example 0.5 to 25 mg such as 0.5, 1, 2, 5, 10, 15 or 20 mg. Such compositions may be administered from 1 to 6 times a day, more usually from 1 to 4 times a day, in a manner such that the daily dose is from 0.01 to 25 mg per kg body weight and more particularly from 0.1 to 10 mg/kg.

No toxicological effects are indicated at the aforementioned dosage ranges.

It will be appreciated that the compounds of the invention may be administered in combination with other antihypertensives, especially ACE inhibitors or $\beta$-blockers, or with diuretics.

The present invention further provides a compound of formula (I) or a pharmaceutically acceptable salt thereof for use as an active therapeutic substance, in particular, in the treatment or prophylaxis of hypertension and/or respiratory tract disorders.

The invention also provides the use of a compound of formula (I) or a pharmaceutically acceptable salt thereof in the manufacture of a medicament for use in the treatment or prophylaxis of hypertension and/or respiratory tract disorders.

Tables 1, 2, 3 and 4 show the intermediates and compounds of the invention prepared.

The following descriptions relate to the preparation of intermediates and the following examples relate to the preparation of compounds of formula (I).

Table 1

Intermediates of formula (VI)

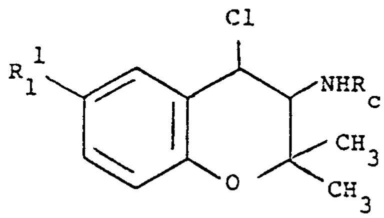

| Description No. | $R_1{}^1$ | $R_c$ |
|---|---|---|
| D1 | NC | $CO_2{}^tBu$ |
| D4 | Cl | $CO_2{}^tBu$ |
| D8 | $F_3C$ | $CO_2{}^tBu$ |
| D13 | NC | $CO_2CH_2Ph$ |

Table 2

Intermediates of formula (V)

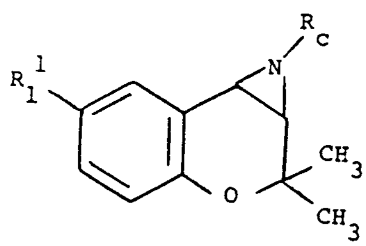

| Description No. | $R_1{}^1$ | $R_c$ |
|---|---|---|
| D2 | NC | $CO_2{}^tBu$ |
| D5 | Cl | $CO_2{}^tBu$ |
| D9 | $F_3C$ | $CO_2{}^tBu$ |
| D14 | NC | $CO_2CH_2Ph$ |

Table 3

Intermediates of formula (II)

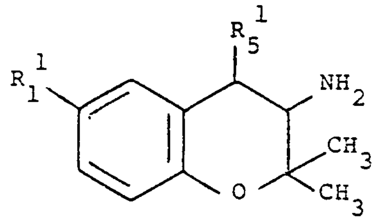

| Description No. | $R_1^1$ | $R_5^1$ |
|---|---|---|
| D3 | NC | OH |
| D7 | Cl | OH |
| D11 | $F_3C$ | OH |

Table 4

Compounds of formula (I)

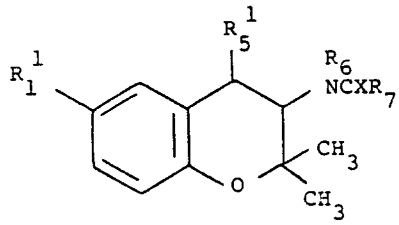

| Example No. | X | $R_1^1$ | $R_5^1$ | $R_6$ | $R_7$ |
|---|---|---|---|---|---|
| E1 | O | NC | OH | H | $O^tBu$ |
| E2 | O | NC | OH | $-(CH_2)_4-$ | |
| E3 | O | NC | OH | H | 4-FPh |
| E4 | O | NC | OH | H | $O^tBu$ |
| E5 | O | NC | OH | H | $NH^tBu$ |
| E6 | O | NC | OH | H | 3,4-FPh |
| E7 | O | NC | OH | H | $NHCH_3$ |
| E8 | O | NC | OH | H | $CH_3$ |
| E9 | O | NC | OH | H | 2-pyridyl |
| E10 | O | NC | OH | H | $CH_2{}^tBu$ |
| E11 | O | NC | OH | H | cyclohexyl |
| E12 | O | NC | OH | H | NH-cyclohexyl |
| E13 | O | NC | OH | H | Ph |
| E14 | O | NC | OH | H | $NHCH(CH_3)_2$ |
| E15 | O | NC | OH | H | $NHCH_2CH_3$ |
| E16 | O | NC | OH | H | $NH(CH)_2CH_3$ |
| E17 | O | Cl | OH | H | $NH^tBu$ |
| E18 | O | NC | OH | H | $N(CH_3)_2$ |
| E19 | S | NC | OH | H | $NHCH(CH_3)_2$ |
| E20 | O | $F_3C$ | OH | H | $NH^tBu$ |
| E21 | O | NC | OH | H | $C(CH_3)_3$ |
| E22 | O | NC | OH | H | $CH(CH_3)_2$ |
| E23 | O | NC | OH | H | $OCH_2Ph$ |
| E24 | O | NC | H | H | $NH^tBu$ |
| E25 | O | NC | $OCH_3$ | H | $O^tBu$ |
| E26 | O | NC | $NH_2$ | H | $O^tBu$ |

Description 1

Trans-6-cyano-3,4-dihydro-2,2-dimethyl-3-(t-butoxycarbonylamino)-4-chloro-2H-1-benzopyran (D1)

A solution of t-butyl-N,N-dichlorocarbamate (2.19 g, prepared by the method of R.E. White and P. Kovacic, J.A.C.S., 97, 1180(1975)) in dry toluene (10 ml) was added dropwise to a solution of 2,2-dimethyl-6-cyano-2H-1-benzopyran (2.0 g) in dry toluene (20 ml) at 35-40°C. The solution was then stirred at 45-50°C for 6 h, cooled to 0°C, and a 20% solution of sodium metabisulphite (20 ml) added. The mixture was stirred vigorously for 18 h and the layers were separated. The organic layer was washed with water, saturated sodium bicarbonate solution, brine, then dried over sodium sulphate. Removal of solvents in vacuo and trituration of the residue with pentane:ethyl acetate (95:5) gave the title compound as a colourless solid (2.1 g, 58%).

A sample recrystallised from ethyl acetate:60-80°C petrol had m.pt. 164-166°C;
$^1H$ n.m.r ($CDCl_3$) $\delta$ 1.30 (s, 3H); 1.46 (s, 9H); 1.53 (s, 3H); 4.17 (t, J = 8, 8Hz, 1H); 4.63 (d, J = 8Hz, 1H); 4.88 (d, J = 8Hz, 1H); 6.92 (d, J = 9Hz, 1H); 7.48 (dd, J = 9, 2Hz, 1H); 7.82 (d, J = 2Hz, 1H).
Anal. Found: C,60.71%, H,6.46%, N,8.33%.
$C_{17}H_{21}N_2O_3Cl$ requires: C,60.62%, H,6.28%, N,8.32%.

Description 2

6-Cyano-3,4-[(N-t-butoxycarbonyl)aziridino]-2,2-dimethyl-2H-1-benzopyran (D2)

A solution of trans-6-cyano-3,4-dihydro-2,2-dimethyl-3-(t-butoxycarbonylamino)-4-chloro-2H-1-benzopyran (1.57 g) and potassium carbonate (1.0 g) in ethanol (100 ml) and water (5 ml) was stirred for 18 h. Solvents were removed in vacuo; and the residue extracted with ethyl acetate. The extracts were dried over magnesium sulphate and evaporated in vacuo to give the title compound (1.38 g, 98% as a colourless solid having m.pt. 116-118°C;
$^1H$ n.m.r. ($CDCl_3$) $\delta$ 1.25 (s, 3H); 1.46 (s, 9H); 1.65 (s, 3H); 3.05 (d, J = 6Hz, 1H); 3.50 (d, J = 6Hz, 1H); 6.86 (d, J = 9Hz, 1H); 7.49 (dd, J = 9, 2Hz, 1H); 7.65 (d, J = 2Hz, 1H).

Description 3

trans-6-Cyano-2,2-dimethyl-3,4-dihydro-3-amino-2H-1-benzopyran-4-ol (D3)

A solution of trans-6-cyano-2,2-dimethyl-3,4-dihydro-3-(t-butoxycarbonyl)amino-2H-1-benzopyran-4-ol (2.0g) (E1), and trifluoroacetic acid (18 ml) in dry dichloromethane (50 ml) was stirred at 0°C for 8h. Solvents were removed in vacuo, and the residue dissolved in ethyl acetate and washed with saturated sodium bicarbonate solution, then dried ($MgSO_4$) and evaporated to give the title compound (1.0g) as a solid which was used without further purification.

$^1H$ n.m.r. ($CDCl_3$) $\delta$:

1.30 (s, 3H); 1.50 (s, 3H); 3.15 (d, J = 9, 1H); 4.40 (brs, 3H); 4.65 (d, J = 9, 1H); 6.72 (d, J = 9, 1H); 7.36 (dd, J = 9,2, 1H); 7.68 (d, J = 2, 1H).

Description 4

trans-6-Chloro-3,4-dihydro-2,2-dimethyl-3-(t-butoxycarbonyl)amino-4-chloro-2H-1-benzopyran (D4)

The compound of this description was prepared from 6-chloro-2,2-dimethyl-2H-1-benzopyran [J.Med.Chem., 1983, 26, 1582], in a similar manner to the compound of description 1, to give the title compound (D4), as a colourless solid having m.pt. 140-1°C.

$^1H$ n.m.r. ($CDCl_3$) $\delta$ 1.30 (s,3H), 1.45 (s,9H), 1.62 (s,3H), 4.16 (m,1H), 4.68 (d,J = 8Hz,1H), 4.87 (d,J = 6Hz,1H), 6.78 (d,J = 9Hz,1H), 7.18 (J = 9,2,Hz,1H), 7.45 (d,J = 2Hz,1H).

Description 5

6-Chloro-3,4-[(N-t-butoxycarbonyl)aziridino]-2,2-dimethyl-2H-1-benzopyran (D5)

The compound of this description was prepared from trans-6-chloro-3,4-dihydro-2,2-dimethyl-3-(t-butoxycarbonylamino)-4-chloro-2H-1-benzopyran (D4), in a similar manner to the compound of description 2, to give the title compound (D5) as a solid, which was used without further purification.

$^1$H n.m.r. ($CDCl_3$) $\delta$ 1.20 (s,3H), 1.45 (s,9H), 1.62 (s,3H), 3.00 (d,J = 6Hz,1H), 3.44 (d,J = 6Hz,1H), 6.75 (d,J = 9Hz,1H), 7.15 (dd,J = 9,2Hz,1H), 7.32 (d,J = 2Hz,1H).

Description 6

trans-6-Chloro-2,2-dimethyl-3,4-dihydro-3-(t-butoxycarbonyl)amino-2H-1-benzopyran-4-ol (D6)

The compound of this description was prepared from 6-chloro-3,4-[(N-t-butoxycarbonyl)aziridino]-2,2-dimethyl-2H-1-benzopyran (D5), in a similar manner to the compound of example 1, to give the title compound as a colourless solid having m.pt. 132-3°C.

$^1$H n.m.r. ($CDCl_3$) $\delta$ 1.25 (s,3H), 1.42 (s,3H), 1.46 (s,9H), 3.83 (m,1H), 4.48 (d,J = 6Hz,1H), 4.66 (m,1H), 4.80-5.20 (brs,1H), 6.74 (d,J = 9Hz,1H), 7.14 (dd,J = 9,2Hz,1H), 7.46 (d,J = 2Hz,1H).

Description 7

trans-6-Chloro-2,2-dimethyl-3,4-dihydro-3-amino-2H-1-benzopyran-4-ol (D7)

The compound of this description was prepared from trans-6-chloro-2,2-dimethyl-3,4-dihydro-3-(t-butoxycarbonyl)amino-2H-1-benzopyran-4-ol (D6), in a similar manner to the compound of description 3, to give the title compound (D7) as a gum.

$^1$H n.m.r. ($CDCl_3$) $\delta$ 1.32 (s,3H), 1.55 (s,3H), 3.26 (d,J = 9Hz,1H), 4.82 (d,J = 9Hz,1H), 5.10-6.00 (brs,3H), 6.72 (d,J = 9Hz,1H), 7.13 (dd,J = 9,2Hz,1H), 7.49 (d,J = 2Hz,1H).

Description 8

trans-6-Trifluoromethyl-3,4-dihydro-2,2-dimethyl-3-(t-butoxycarbonyl)amino-4-chloro-2H-1-benzopyran (D8)

The compound of this description was prepared from 6-trifluoromethyl-2,2-dimethyl-2H-1-benzopyran, in a similar manner to the compound of description 1, to give the title compound (D8) which recrystallised from ethyl acetate: 60-80°C petrol as colourless needles having m.pt. 220-3°C (decomp.).

$^1$H n.m.r. ($CDCl_3$) $\delta$ 1.30 (s,3H), 1.46 (s,9H), 1.54 (s,3H), 4.19 (dd,J = 9,6Hz,1H), 4.67 (d,J = 9Hz,1H), 4.92 (d,J = 6Hz,1H), 6.92 (d,J = 9Hz,1H), 7.46 (dd,J = 9,2Hz,1H), 7.75 (d,J = 2Hz,1H).

Description 9

6-Trifluoromethyl-3,4-[(N-t-butoxycarbonyl)aziridino]-2,2-dimethyl-2H-1-benzopyran (D9)

The compound of this description was prepared from trans-6-trifluoromethyl-3,4-dihydro-2,2-dimethyl-3-(t-butoxycarbonyl)amino-4-chloro-2H-1-benzopyran (D8), in a similar manner to the compound of description 2, to give the title compound (D9) as a solid, which was used without further purification.

$^1$H n.m.r. ($CDCl_3$) $\delta$ 1.25 (s,3H), 1.45 (s,9H), 1.60 (s,3H), 3.02 (d,J = 6Hz,1H), 3.50 (d,J = 6Hz,1H), 6.85 (d,J = 9Hz,1H), 7.40-7.65 (m,2H).

Description 10

trans-6-Trifluoromethyl-2,2-dimethyl-3,4-dihydro-3-(t-butoxycarbonyl)amino-2H-1-benzopyran-4-ol (D10)

The compound of this description was prepared from 6-trifluoromethyl-3,4-[(N-t-butoxycarbonyl)-aziridino]-2,2-dimethyl-2H-1-benzopyran (D9), in a similar manner to the compound of example 1, to give the title compound (D10) as colourless microcrystals having m.pt. 147-8°C.

$^1H$ n.m.r. ($CDCl_3$) δ 1.27 (s,3H), 1.44 (s,9H), 1.48 (s,3H), 2.80-3.30 (brs,1H), 3.88 (m,1H), 4.54 (d,J=8Hz,1H), 4.65 (m,1H), 6.89 (d,J=9Hz,1H), 7.44 (dd,J=9,2Hz,1H), 7.28 (d,J=2Hz,1H).

Description 11

trans-6-Trifluoromethyl-2,2-dimethyl-3,4-dihydro-3-amino-2H-1-benzopyran-4-ol (D11)

The compound of this description was prepared from trans-6-trifluoromethyl-2,2-dimethyl-3,4-dihydro-3-(t-butoxycarbonyl)amino-2H-1-benzopyran-4-ol (D10),in a similar manner to the compound of description 3, to give the title compound (D11) as a crude solid, which was used without further purification.

$^1H$ n.m.r. ($CDCl_3$) δ 1.20 (s,3H), 1.48 (s,3H), 2.20-2.30 (brs,3H), 2.87 (d,J=10Hz,1H), 4.40 (d,J=10Hz,1H), 6.85 (d,J=9Hz,1H), 7.43 (dd,J=9,2Hz,1H), 7.78 (d,J=2Hz,1H).

Description 12

Benzyl-N,N-dichlorocarbamate (D12)

Hydrochloric acid (54 mL, 5N) was added dropwise to a stirred suspension of benzylcarbamate (9.6g) and calcium hypochlorite (18.3g) in dichloromethane (80 mL) at 0°C. The mixture was stirred whilst allowing to warm to room temperature over 1h, separated and the organic layer washed with water, then dried ($MgSO_4$). Evaporation of solvent in vacuo gave the title compound (D13) as a gum (13.0g) which was used without further purification.

$^1H$ n.m.r. ($CDCl_3$) δ 5.24 (s,2H), 7.35 (s,5H).

Description 13

trans-6-Cyano-3,4-dihydro-2,2-dimethyl-3-(benzyloxycarbonylamino)-4-chloro-2H-1-benzopyran (D13)

The compound of this description was prepared from 2,2-dimethyl-6-cyano-2H-1-benzopyran in a similar manner to the compound of description 1, but using benzyl-N,N-dichlorocarbamate (D13). Recrystallisation from ethyl acetate:60-80°C petrol gave the title compound (D13) as colourless needles having m.pt. 125-6°C.

$^1H$ n.m.r. ($CDCl_3$) δ 1.30 (s,3H), 1.53 (s,3H), 4.25 (m,1H), 4.80-5.00 (m,1H) overlapping 4.90 (d,J=6Hz,1H), 5.18 (s,2H), 6.90 (d,J=9Hz,1H), 7.36 (s,5H), 7.48 (dd,J=9,2Hz,1H), 7.80 (d,J=2Hz,1H).

Description 14

6-Cyano-3,4-[(N-benzyloxycarbonyl)aziridino]-2,2-dimethyl-2H-1-benzopyran (D14)

A mixture of trans-6-cyano-3,4-dihydro-2,2-dimethyl-3-(benzyloxycarbonyl)amino-4-chloro-2H-1-benzopyran (D13) (0.5g), potassium bicarbonate (0.25g), and potassium carbonate (50 mg) in ethanol (20 mL) and water (0.5 mL) was stirred vigorously for 4 days. Solvents were removed in vacuo, and the residue extracted with ethyl acetate. The extracts were dried ($MgSO_4$) and evaporated in vacuo to give the title compound (D14) (0.35g) as a solid which was used without further purification.

$^1H$ n.m.r. ($CDCl_3$) δ 1.25 (s,3H), 1.55 (s,3H), 3.15 (d,J=6Hz,1H), 3.60 (d,J=6Hz,1H), 5.15 (s,2H), 6.82 (d,J=9Hz,1H), 7.30 (s,5H), 7.45-7.80 (m,2H).

Description 15

trans-6-Cyano-3,4-dihydro-2,2-dimethyl-3-(t-butoxycarbonyl)amino-4-azido-2H-1-benzopyran (D15)

Sodium azide (0.79g) was added to a stirred mixture of 6-cyano-3,4-[(N-t-butoxycarbonyl)aziridino]-2,2-dimethyl-2H-1-benzopyran (D2) (3.32g), and ammonium chloride (0.64g) in dry N,N-dimethylformamide (30mL). The reaction mixture was heated at 60-65°C for 5h, then cooled and partitioned between ethyl acetate and water. The organic layer was washed with water, brine and then dried ($HgSO_4$) and evaporated in vacuo to give the title compound (D15) (2.9g). A sample recrystallised from ethyl acetate: 60-80°C petrol having mpt 167-8°C.

Example 1

trans-6-Cyano-2,2-dimethyl-3,4-dihydro-3-(t-butoxycarbonyl)amino-2H-1-benzopyran-4-ol (E1)

A solution of 6-cyano-3,4-[N-t-butoxycarbonyl)-aziridino]-2,2-dimethyl-2H-1-benzopyran (D2) (0.5g) in dioxan (20 ml) and water (20 ml) was treated with dil. $H_2SO_4$ (0.25 ml) and the solution stirred at room temperature for 18h. Solvents were evaporated in vacuo and the residue partitioned between ethyl acetate and water. The organic extracts were dried ($MgSO_4$) and evaporated to give a solid which recrystallised from 60-80°C petrol:ethyl acetate as microcrystals (0.2g) having m.pt. 150-2°C.

$^1H$ n.m.r. ($CDCl_3$) $\delta$:

1.25 (s, 3H); 1.42 (s, 9H); 1.46 (s, 3H); 3.50 (br s, OH); 3.85 (m, 1H); 4.52 (d, J=9, 1H); 4.70 (d, NH); 6.87 (d, J=9, 1H); 7.46 (dd, J=9,2, 1H); 7.82 (d, J=2, 1H).

Found: C, 64.30; H, 7.14; N, 8.79%; $C_{17}H_{22}N_2O_4$
requires C, 64.13; H, 6.97; N, 8.80%.

Example 2

trans-6-Cyano-2,2-dimethyl-3,4-dihydro-3-(2-oxo-1-piperidinyl)-2H-1-benzopyran-4-ol (E2)

To a solution of trans-6-cyano-2,2-dimethyl-3,4-dihydro-3-amino-2H-1-benzopyran-4-ol (D3) (1.0g) and triethylamine (1 ml) in dichloromethane (30 ml) at 0°C was added 5-chlorovalerylchloride (0.71g). The solution was stirred at room temperature for 24h, then water was added, and the mixture extracted with ethyl acetate. The combined organic layers were washed with dil. HCl, water, brine, and dried ($MgSO_4$). Removal of solvents in vacuo gave the crude amide (1.2g) which was dissolved in dry tetrahydrofuran (100 ml) and sodium hydride (0.116g, 80% dispersion in oil) added. The solution was stirred at room temperature for 6 days, then water was added, and the mixture extracted with ethyl acetate. The combined organic layers were dried ($MgSO_4$) and evaporated in vacuo. The residue was chromatographed on a silica gel column eluted with ethyl acetate to give the title compound (0.2g) which recrystallised from ethyl acetate:pentane as needles having m.pt. 222-5°C.

$^1H$ n.m.r. (DMSO-$d_6$) $\delta$:

1.32 (s, 3H); 1.42 (s, 3H); 1.80 (m, 4H); 2.45 (m, 2H); 3.28 (m, 2H); 3.50 (brs, 1H); 4.78 (d, J=8, 1H); 5.10 (d, J=8, 1H); 6.86 (d, J=9, 1H); 7.46 (dd, J=9,2, 1H); 7.85 (d, J=2, 1H).

Example 3

trans-6-Cyano-2,2-dimethyl-3,4-dihydro-3-(4-fluorobenzoylamino)-2H-1-benzopyran-4-ol (E3)

To a solution of trans-6-cyano-2,2-dimethyl-3,4-dihydro-3-amino-2H-1-benzopyran-4-ol (D3) (0.576g) and triethylamine (1 ml) in dichloromethane (40 ml) at 0°C, was added 4-fluorobenzoyl chloride (0.835g). The reaction mixture was stirred at room temperature for 3 days, then water was added, and the mixture separated. The organic layer was washed with dil. HCl, sodium bicarbonate solution, water, brine, and then dried. Solvent removed in vacuo, and the residue dissolved in methanol (20 ml) and sodium hydroxide solution (0.4 ml, 10%) added. The solution was stirred at room temperature for 18h, then water was added and the mixture extracted with ethyl acetate. The combined organic extracts were washed with water, and dried ($MgSO_4$). Evaporation of solvent in vacuo gave a solid which recrystallised from ethyl acetate: 60-80°C petrol as colourless microcrystals (0.2g) having m.pt. 212-5°C.

$^1H$ n.m.r. ($CDCl_3$) $\delta$:

1.34 (s, 3H); 1.50 (s, 3H); 3.35 (brs, 1H); 3.90 (brs, 1H); 4.40 (d, J=9, 1H); 4.70 (d, J=9, 1H); 6.90 (d, J=9, 1H); 7.15 (m, 2H); 7.50 (m, 1H); 7.90 (m, 3H).

Example 4

2,2-Dimethyl-3,4-dihydro-3-(t-butoxycarbonyl)amino-6-cyano-2H-1-benzopyran (E4)

A solution of 6-cyano-2,2-dimethyl-2H-1-benzopyran (1.0g) and N,N-dichloro-t-butylurethane (1.1g) in toluene (20 ml) was heated at 50°C for 5 h. The solution was cooled, and solvent removed in vacuo. The residue was dissolved in dioxan (20 ml) at 0°C, a solution of ammonium acetate (2.08g) in water (20 ml) added, and zinc dust (1.76g) added portionwise. The mixture was stirred for 18 h at room temperature,

decanted, and diluted with water and extracted with ethyl acetate. The organic extracts were washed with brine, dried ($MgSO_4$) and evaporated in vacuo. The residue was chromatographed on a silica gel column eluted with ethyl acetate:pentane (1:9) to give the title compound as a solid (1.17g, 72%) which recrystallised from 60-80° petrol as needles having m.pt. 141-2°C.
$^1$H n.m.r. ($CDCl_3$) δ 1.32 (s, 3H); 1.38 (s, 3H); 1.45 (s, 9H); 2.70 (dd, J = 15, 4, 1H); 3.13 (dd, J = 15, 4, 1H); 3.97 (m, 1H); 4.58 (d, J = 9, NH); 6.87 (d, J = 9, 1H); 7.40 (m, 2H).

Example 5

trans-6-Cyano-2,2-dimethyl-3,4-dihydro-3-(t-butylureido)-2H-1-benzopyran-4-ol (E5)

A solution of t-butylisocyanate (0.14g) in dichloromethane (10ml) was added to a stirred solution of trans-6-cyano-2,2-dimethyl-3,4-dihydro-3-amino-2H-1-benzopyran-4-ol (0.303g) (D3) in dichloromethane (30ml) at 0°C. The solution was allowed to warm to room temperature, and then stirred for a further 1 hr. The precipitate was collected by filtration to give the required compound (0.2g, 46%) as a colourless solid having m.pt. 129-132°C.

$^1$H n.m.r. (DMSO-$d^6$) δ: 1.27 (s,3H), 1.32 (s,9H), 1.42 (s,3H), 3.88 (m,1H), 4.40 (apparent t, J = 9, 1H), 5.82 (br s, NH), 5.88 (d, J = 6, OH), 5.95 (d,J = 9,NH), 7.04 (d,J = 9,1H), 7.70 (dd, J = 9,2,1H), 7.89 (d,J = 2,1H).

Example 6

trans-6-Cyano-2,2-dimethyl-3,4-dihydro-3-(3,4-difluorobenzoylamino)-2H-1-benzopyran-4-ol (E6)

The compound of this example was prepared from D3 in a similar manner to the compound of Example 3, but using 3,4-difluorobenzoyl chloride. Recrystallisation from ethyl acetate: 60-80°C petrol gave the title compound (E6) as colourless microcrystals having m.pt. 220-222°C.
Anal. Found: C,63.64, H,4.53, N,7.70%; $C_{19}H_{16}N_2O_3F_2$
requires C,63.68, H,4.50, N,7.82%.

Example 7

trans-6-Cyano-2,2-dimethyl-3,4-dihydro-3-(methylureido)-2H-1-benzopyran-4-ol (E7)

The compound of this example was prepared from D3 in a similar manner to the compound of Example 5, but using methylisocyanate to give the title compound (E7) as a colourless solid, having m.pt. 217°C (decomp.).

$^1$H n.m.r. (DMSO-$d_6$) δ 1.25 (s,3H), 1.47 (s,3H), 2.74 (s,3H), 3.30-3.60 (brs,2H), 3.95 (m,1H), 4.45 (d,J = 9Hz,1H), 5.90 (d,J = 8Hz,1H), 6.85 (d,J = 9Hz,1H), 7.43 (dd,J = 9,2Hz,1H), 7.82 (d,J = 2Hz,1H).

Example 8

trans-6-Cyano-2,2-dimethyl-3,4-dihydro-3-acetylamino-2H-1-benzopyran-4-ol (E8)

The compound of this example was prepared from D3 in a similar manner to the compound of Example 3, but using acetyl chloride. Recrystallisation from ethyl acetate: 60-80°C petrol gave the title compound (E8) as a colourless solid having m.pt. 179-181°C.
Anal. Found: C,64.60, H,6.24, N,10.54%; $C_{14}H_{16}N_2O_3$
requires C,64.60, H,6.20, N,10.76%.

Example 9

trans-6-Cyano-2,2-dimethyl-3,4-dihydro-3-(2-pyridine-carbonylamino)-2H-1-benzopyran-4-ol (E9)

Ethyl chloroformate (0.6g) in dichloromethane (10 mL) was added dropwise to a solution of picolinic acid (0.68g) in dichloromethane (50 mL) and triethylamine (0.77 mL), and the solution stirred at room temperature for 2h. A solution of trans-6-cyano-2,2-dimethyl-3,4-dihydro-3-amino-2H-1-benzopyran-4-ol (1.0g) (D3) in dichloromethane (30 mL) was then added, and the reaction mixture stirred at room

temperature for 48h. Water was then added, the layers separated, and the organic layer washed with water, brine, then dried ($MgSO_4$) and evaporated in vacuo. The residue was triturated with ethyl acetate:pentane (1:1) and recrystallised from ethyl acetate:methanol:60-80°C petrol to give the title compound (E9) (300 mg) as a colourless solid having m.pt. 216-9°C.

$^1$H n.m.r. (DMSO-$d_6$) δ 1.35 (s,3H), 1.47 (s,3H), 4.33 (t,J=9Hz,1H), 4.96 (dd,J=9,7Hz,1H), 5.90 (d,J=7Hz,1H), 7.08 (d,J=9Hz,1H), 7.75 (m,2H), 7.92 (m,1H), 8.10-8.20 (m,2H), 8.80 (m,2H).

Example 10

trans-6-Cyano-2,2-dimethyl-3,4-dihydro-3-(t-butylacetylamino)-2H-1-benzopyran-4-ol (E10)

The compound of this example was prepared from D3 in a similar manner to the compound of Example 3, but using t-butylacetyl chloride. Recrystallisation from ethyl acetate:60-80°C petrol gave the title compound (E10) as a colourless solid having m.pt. 174-6°C.

Anal. Found: C,68.23, H,7.65, N,8.68%; $C_{18}H_{24}N_2O_3$
requires C,68.33, H,7.65, N,8.85%.

Example 11

trans-6-Cyano-2,2-dimethyl-3,4-dihydro-3-(cyclohexane-carbonylamino)-2H-1-benzopyran-4-ol (E11)

The compound of this example was prepared from D3 in a similar manner to the compound of Example 3, but using cyclohexanecarbonyl chloride. Recrystallisation from ethyl acetate:60-80°C petrol gave the title compound (E11) as a colourless solid having m.pt. 208-10°C.

$^1$H n.m.r. ($CDCl_3$) δ 1.10-1.40 (m,4H), overlapping 1.30 (s,3H), 1.44 (s,3H), 1.60-1.85 (m,6H), 2.10 (m,1H), 3.30-3.70 (brs,1H), 4.21 (dd,J=10,8Hz,1H), 4.50 (d,J=8Hz,1H), 5.53 (d,J=10Hz,1H), 6.89 (d,J=9Hz,1H), 7.47 (dd,J=9,2Hz,1H), 7.83 (d,J=2Hz,1H).

Example 12

trans-6-Cyano-2,2-dimethyl-3,4-dihydro-3-(cyclohexylureido)-2H-1-benzopyran-4-ol) (E12)

The compound of this example was prepared from D3 in a similar manner to the compound of Example 5, but using cyclohexylisocyanate, to give the title compound (E12) as a colourless solid having m.pt. 182-3°C.

Anal. Found: C,66.46, H,7.45, N,12.35%; $C_{19}H_{25}N_3O_3$
requires C,66.65, H,7.07, N,12.27%.

Example 13

trans-6-Cyano-2,2-dimethyl-3,4-dihydro-3-benzoylamino-2H-1-benzopyran-4-ol (E13)

The compound of this example was prepared from D3 in a similar manner to the compound of Example 3, but using benzoyl chloride. Recrystallisation from ethyl acetate:60-80°C petrol gave the title compound (E13) as colourless plates having m.pt. 183-5°C.

$^1$H n.m.r. (DMSO-$d_6$) δ 1.25 (s,3H), 1.38 (s,3H), 4.27 (t,J=8Hz,1H), 4.75 (dd,J=8,6Hz,1H), 5.79 (d,J=6Hz,1H), 6.95 (d,J=9Hz,1H), 7.45-7.65 (m,4H), 7.85-7.95 (m,3H), 8.42 (d,J=8Hz,1H).

Example 14

trans-6-Cyano-2,2-dimethyl-3,4-dihydro-3-(isopropylureido)-2H-1-benzopyran-4-ol (E14)

The compound of this example was prepared from D3 in a similar manner to the compound of Example 5, but using isopropylisocyanate. Recrystallisation from methanol:ethyl acetate:60-80°C petrol gave the title compound (E14) as a colourless solid having m.pt. 198-200°C.

$^1$H n.m.r. (DMSO-$d_6$) δ 1.12 (s,3H), 1.15 (s,3H), 1.36 (s,3H), 1.43 (s,3H), 3.77 (m,1H), 3.90 (m,1H), 4.43 (m,1H), 5.80 (d,J=6Hz,1H), 5.86 (d,J=6Hz,1H), 5.97 (d,J=10Hz,1H), 7.02 (d,J=9Hz,1H), 7.70 (dd,J=9,2Hz,1H), 7.90 (d,J=2Hz,1H).

Example 15

trans-6-Cyano-2,2-dimethyl-3,4-dihydro-3-(ethylureido)-2H-1-benzopyran-4-ol (E15)

The compound of this example was prepared from D3 in a similar manner to the compound of Example 5, but using ethylisocyanate. Recrystallisation from ethyl acetate:60-80°C petrol gave the title compound (E15) as a colourless solid having m.pt. 173-5°C.

Example 16

trans-6-Cyano-2,2-dimethyl-3,4-dihydro-3-(n-propylureido)-2H-1-benzopyran-4-ol) (E16)

The compound of this example was prepared from D3 in a similar manner to the compound of Example 5, but using n-propylisocyanate. Recrystallisation from methanol:ethyl acetate:60-80°C petrol gave the title compound (E16) as a colourless solid having m.pt. 190-2°C.

Example 17

trans-6-Chloro-2,2-dimethyl-3,4-dihydro-3-(t-butylureido)-2H-1-benzopyran-4-ol (E17)

The compound of this example was prepared from trans-6-chloro-2,2-dimethyl-3,4-dihydro-3-amino-2H-1-benzopyran-4-ol (D7), in a similar manner to the compound of Example 5. Recrystallisation from ethyl acetate:60-80°C petrol gave the title compound (E17) as colourless crystals having m.pt. 190-3°C.

Example 18

trans-6-Cyano-2,2-dimethyl-3,4-dihydro-3-(dimethylaminocarbonylamino)-2H-1-benzopyran-4-ol (E18)

A solution of trans-6-cyano-2,2-dimethyl-3,4-dihydro-3-amino-2H-1-benzopyran-4-ol (500 mg) (D3), triethylamine (1 mL), and N,N-dimethylcarbamyl chloride (247 mg) in dichloromethane (30 mL) was stirred at room temperature for 42 days. Dilute hydrochloric acid (50 mL, 2N) was then added, and the layers separated. The organic layer was washed with sodium bicarbonate solution, brine, then dried ($Na_2SO_4$) and evaporated in vacuo. The residue was chromatographed (Si gel, eluant EtOAc:pentane) to give the crude product which was recrystallised from ethyl acetate:methanol as colourless plates (200 mg) having m.pt. 210-212°C.
Anal. Found: C,62.35, H,6.63, N,14.50%; $C_{15}H_{19}N_3O_3$
requires C,62.28, H,6.57, N,14.53%.

Example 19

trans-6-Cyano-2,2-dimethyl-3,4-dihydro-3-(isopropylthioureido)-2H-1-benzopyran-4-ol (E19)

The compound of this example was prepared from D3 in a similar manner to the compound of Example 5, but using isopropylthioisocyanate, to give the title compound as a colourless solid having m.pt. 100-102°C (decomp.).
$^1$H n.m.r. ($CDCl_3$) $\delta$ 1.21 (s,3H), 1.25 (s,3H), 1.35 (s,3H), 1.51 (s,3H), 2.60-3.30 (brs,1H), 4.20 (m,1H), 4.60 (m,1H), 4.77 (d,J=6Hz,1H), 6.04 (brs,1H), 6.25 (brs,1H), 6.90 (d,J=9Hz,1H), 7.47 (dd,J=9,2Hz,1H), 7.75 (d,J=2Hz,1H).

Example 20

trans-6-Trifluoromethyl-2,2-dimethyl-3,4-dihydro-3-(t-butylureido)-2H-1-benzopyran-4-ol (E20)

The compound of this example was prepared from trans-6-trifluoromethyl-2,2-dimethyl-3,4-dihydro-3-amino-2H-1-benzopyran-4-ol (D11), in a similar manner to the compound of Example 5. Recrystallisation from ethyl acetate:60-80°C petrol gave the title compound (E20) as colourless microcrystals having m.pt. 193-5°C(decomp.).

Example 21

trans-6-Cyano-2,2-dimethyl-3,4-dihydro-3-trimethylacetylamino-2H-1-benzopyran-4-ol (E21)

The compound of this example was prepared from D3 in a similar manner to the compound of Example 3, but using trimethylacetyl chloride. Recrystallisation from ethyl acetate:60-80°C petrol gave the title compound (E21) as a colourless solid having m.pt. 186-8°C.

$^1$H n.m.r. ($CDCl_3$) δ 1.16 (s,9H), 1.30 (s,3H), 1.42 (s,3H), 4.23 (dd,J = 9,8Hz,1H), 4.50 (d,J = 8Hz,1H), 5.70 (d,J = 9Hz,1H), 6.89 (d,J = 9Hz,1H), 7.48 (dd,J = 9,2Hz,1H), 7.84 (d,J = 2Hz,1H).

Example 22

trans-6-Cyano-2,2-dimethyl-3,4-dihydro-3-isobutyrylamino-2H-1-benzopyran-4-ol (E22)

The compound of this example was prepared from D3 in a similar manner to the compound of Example 3, but using isobutyryl chloride. Recrystallisation from ethyl acetate:60-80°C petrol gave the title compound (E24) as a colourless solid having m.pt.176-8°C.

Example 23

trans-6-Cyano-3,4-dihydro-2,2-dimethyl-3-(benzyloxycarbonyl)amino-2H-1-benzopyran-4-ol (E23)

The compound of this example was prepared in a similar manner, from 6-cyano-3,4-[(N-benzyloxycarbonyl)-aziridino]-2,2-dimethyl-2H-1-benzopyran (D14) in a similar manner to the compound of Example 1. Recrystallisation from ethyl acetate:60-80°C petrol gave the title compound (E23) as a colourless solid having m.pt. 174-7°C.

Example 24

2,2-Dimethyl-3,4-dihydro-3-(t-butylureido)-6-cyano-2H-1-benzopyran (E24)

A solution of 2,2-dimethyl-3,4-dihydro-3-(t-butoxycarbonyl)amino-6-cyano-2H-1-benzopyran (E4) (0.5g) and trifluoroacetic acid (5 mL) in dry dichloromethane (30 mL) was stirred at 0°C for 5h. Solvents were removed in vacuo, and the residue dissolved in ethyl acetate and washed with sodium bicarbonate solution, water, brine and then dried ($MgSO_4$). Removal of drying agent and solvent gave a gum (0.24 g) which was dissolved in dry dichloromethane (30 mL), cooled to 0°C, under nitrogen, and t-butylisocyanate (0.13g) added. The reaction mixture was stirred at room temperature for 48h, solvent evaporated in vacuo, and the residue chromatographed on silica gel, eluting with 50% ethyl acetate:pentane, to give the title compound (E24) as a solid (0.17g).

$^1$H n.m.r. ($CDCl_3$) δ 1.29 (s,9H), 1.31 (s,3H), 1.39 (s,3H), 2.70 (d,J = 15Hz,1H), 3.12 (dd,J = 15,4Hz,1H), 4.10-4.20 (m,2H), 6.87 (d,J = 9Hz,1H), 7.30 (s,1H), 7.40 (m,2H).

Example 25

trans-6-Cyano-3,4-dihydro-2,2-dimethyl-3-(t-butoxycarbonyl)amino-4-methoxy-2H-1-benzopyran (E25)

To a stirred solution of 6-cyano-3,4-[(N-t-butoxycarbonyl)aziridino]-2,2-dimethyl-2H-1-benzopyran (300 mg) (D2) in dry methanol (30 mL) was added boron trifluoride etherate (0.1 mL) and the solution stirred at room temperature for 18h. Solvents were evaporated in vacuo, and the residue chromatographed on a silica gel column, eluting with 30% ethyl acetate:pentane, to give the crude product which was triturated with pentane:ethyl acetate (9:1) to give the title compound as a colourless solid (90 mg) having m.pt. 121-3°C.

Example 26

trans-6-Cyano-3,4-dihydro-2,2-dimethyl-3-(t-butoxycarbonyl)amino-4-amino-2H-1-benzopyran (E26)

Propane-1,3-dithiol (1.38g) was added to a stirred solution of trans-6-cyano-3,5-dihydro-2,2-dimethyl-3-(t-butoxycarbonyl)amino-4-azido-2H-1-benzopyran (D15) (2.36g) in methanol (40mL) and triethylamine

(1.9mL). The reaction mixture was filtered and solvents removed in vacuo. The residue was dissolved in ether and extracted with 2N HCl (3 x 100mL). The acid extracts were basified with NaOH, and extracted with ethyl acetate (2 x 100mL). The organic extracts were dried ($MgSo_4$) and evaporated in vacuo to give the title compound (E26) as a solid having m.pt. 123-6°C.

## PHARMACOLOGICAL DATA

### 1. Antihypertensive Activity

Systolic blood pressures were recorded by a modification of the tail cuff method described by I.M. Claxton, M.G. Palfreyman, R.H. Poyser, R.L. Whiting, European Journal of Pharmacology, 37, 179 (1976). A W+W BP recorder, model 8005 was used to display pulses. Prior to all measurements rats were placed in a heated environment (33.5 ± 0.5°C) before transfer to a restraining cage. Each determination of blood pressure was the mean of at least 5 readings. Spontaneously hypertensive rats (ages 12-18 weeks) with systolic blood pressures >180 mmHg were considered hypertensive.

The results were as follows:

| Compound | Dose (mg/kg) | % b.p. fall* |
|---|---|---|
| E1 | 3.0 | 30 |
| E3 | 3.0 | 42 |
| E5 | 0.3 | ≧40 |
| E14 | 0.3 | 22 |
| E21 | 1.0 | 36 |

* maximum at 1-4 hours post dose.

### 2. Bronchodilator Activity

Male guinea pigs (300-600g) were stunned by a blow to the head and bled from the carotid artery. The trachea was exposed, dissected free of connective tissue, and transferred to oxygenated Krebs solution at 37°C. Next, spirals (2 per trachea) were prepared by cutting the whole trachea spirally along its longitudinal axis and then dividing this spiral lengthwise. Each preparation was mounted, using silk thread, in a 10ml organ bath filled with krebs solution at 37°C and bubbled with 5% $CO_2$ with $O_2$. The resting tension of the preparations was set at 2g and changes in muscle tension were monitored isometrically by means of a UFI (2oz) force and displacement transducer (Ormed Ltd) connected to a Linseis pen recorder. All preparations were allowed to equilibrate for 60 minutes. During this equilibration period the preparations were washed by upward displacement at 15 minute intervals and, if necessary, the resting tension was readjusted to 2g using a mechanical micromanipulator system.

Once a steady resting tension had been obtained, the preparations were dosed simultaneously with the test compound ($10^{-8}$-$2x10^{-5}$M), and finally a maximum relaxation achieved by addition of $10^{-3}$M isoprenaline. The fall in tension evoked by the test compound was expressed as a percentage of the total relaxation evoked in the presence of $10^{-3}$ isoprenaline. Appropriate concentration-relaxation curves were then constructed and values for potency ($IC_{50}$) were obtained.

[The composition of Krebs solution is: sodium chloride 118.07mM, sodium hydrogen carbonate 26.19mM, potassium chloride 4.68mM, potassium orthophosphate 1.18mM, magnesium sulphate septahydrate 1.8mM and calcium chloride 2.52mM;pH ca. 7.45.]

The compound of Example 5 gave an $IC_{50}$ value of 2.8$\mu$m.

**Claims**

**Claims for the following Contracting States : AT, BE, CH, DE, FR, GB, GR, IT, LI, LU, NL, SE**

1. A compound of formula (I), or a pharmaceutically acceptable salt thereof:

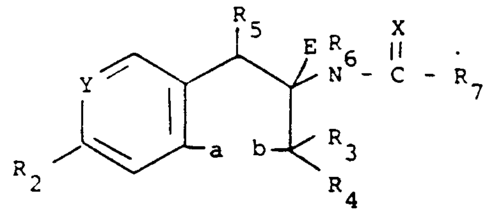

(I)

wherein

a and b together form an -O- linkage or a bond or (when $R_2$ is hydrogen), $CH_2$;

either Y is N and $R_2$ is hydrogen; or

Y is C-$R_1$

wherein

either one of $R_1$ and $R_2$ is hydrogen and the other is nitro, cyano, halo, $CF_3$, $C_2F_5$, formyl, aldoxime, $CF_3O$, $NO_2$-CH=CH-, NC-CH=CH-; a group $R_xX$-wherein $R_x$ is $C_{1-6}$ alkyl, aryl or heteroaryl either of which may be optionally substituted by one, two or three of $C_{1-4}$ alkyl, $C_{1-4}$ alkoxy, nitro, halo, $CF_3$ and cyano; and X is C=O, O.C=O, C=O.O, CHOH, SO, $SO_2$, O.SO, O.$SO_2$, CONH, O.CONH, O.$SO_2$NH, CO-CH=CH, C=NHOH, C=$NNH_2$; or a group $R_yR_zNZ$- wherein $R_y$ and $R_z$ are independently hydrogen or $C_{1-6}$ alkyl and Z is C=O, SO or $SO_2$; or a group $(R_wO)_2P(O)W$ wherein $R_w$ is hydrogen or $C_{1-6}$ alkyl and W is O or a bond; or

$R_1$ is a $C_{3-8}$ cycloalkyl group or a $C_{1-6}$ alkyl group optionally substituted by a group which is hydroxy, $C_{1-6}$ alkoxy, amino optionally substituted by one or two $C_{1-6}$ alkyl groups, $C_{1-7}$ alkanoylamino, $C_{3-8}$ cycloalkyloxy or $C_{3-8}$ cycloalkylamino; and $R_2$ is hydrogen; or

one of $R_1$ and $R_2$ is nitro, cyano or $C_{1-3}$ alkylcarbonyl and the other is a different group selected from nitro, cyano, halo, $C_{1-3}$ alkylcarbonyl, methoxy or amino optionally substituted by one or two $C_{1-6}$ alkyl or by $C_{2-7}$ alkanoyl; or

$R_1$ and $R_2$ together with the carbon atoms to which they are attached, form 2,1,3-oxadiazole;

either one of $R_3$ and $R_4$ is hydrogen or $C_{1-4}$ alkyl and the other is $C_{1-4}$ alkyl; or $R_3$ and $R_4$ together are $C_{2-5}$ polymethylene

$R_5$ is hydrogen, hydroxy, amino, $C_{1-6}$ alkoxy or $C_{1-7}$ acyloxy; and

E is hydrogen; or

$R_5$ and E together form a bond;

either $R_6$ is hydrogen or $C_{1-6}$ alkyl; and

$R_7$ is hydrogen, $C_{3-8}$ cycloalkyl, $C_{2-6}$ alkenyl, $C_{1-6}$ alkyl optionally substituted by hydroxy, $C_{1-6}$ alkoxy, $C_{1-6}$ alkoxycarbonyl, carboxy, halogen or amino optionally substituted by one or two $C_{1-6}$ alkyl groups; aryl or heteroaryl either being optionally substituted by one or more groups or atoms selected from the class of $C_{1-6}$ alkyl, $C_{1-6}$ alkoxy, hydroxy, halogen, trifluoromethyl, nitro, cyano, $C_{1-12}$ carboxylic acyl, or $R_7$ is amino or aminocarbonyl optionally substituted by one or two $C_{1-6}$ alkyl groups or substituted by $C_{3-8}$ cycloalkyl, or a group $OR_9$ wherein $R_9$ is $C_{1-6}$ alkyl or benzyl optionally substituted as defined for $R_7$; or

$R_6$ and $R_7$ are joined together to form $C_{3-4}$ polymethylene optionally substituted by one or two $C_{1-6}$ alkyl groups and optionally containing one or, (when $C_4$ polymethylene), two, C=C double bond(s); and

x is oxygen or sulphur.

2. A compound according to claim 1 wherein Y is C-$R_1$ and a and b together are an -O- linkage.

3. A compound according to claim 1 or 2 wherein $R_1$ is nitro, cyano, acetyl, $CF_3$, $C_2F_5$ or $C_{1-4}$ alkyl, and $R_2$ is hydrogen.

**4.** A compound according to claim 3 wherein $R_1$ is cyano.

**5.** A compound according to any one of claims 1 to 4 wherein $R_3$ and $R_4$ are both methyl groups.

**6.** A compound according to any one of claims 1 to 5 wherein $R_5$ is hydroxy and E is hydrogen and the $R_6NCXR_7$ moiety is trans to $R_5$.

**7.** A compound according to any one of claims 1 to 6 wherein $R_6$ is hydrogen and $R_7$ is optionally substituted phenyl, branched $C_{1-6}$ alkyl, $C_{1-6}$alkoxy, benzyloxy or optionally substituted amino.

**8.** A compound according to claim 7 wherein $R_7$ is t-butylamino.

**9.** A compound selected from the group consisting of:
trans-6-cyano-2,2-dimethyl-3,4-dihydro-3-(t-butoxycarbonyl)amino-2H-1-benzopyran-4-ol,
trans-6-cyano-2,2-dimethyl-3,4-dihydro-3-(2-oxo-1-piperidinyl)-2H-1-benzopyran-4-ol,
trans-6-cyano-2,2-dimethyl-3,4-dihydro-3-(4-fluorobenzoylamino-2H-1-benzopyran-4-ol,
2,2-dimethyl-3,4-dihydro-3-(t-butoxycarbonyl)amino-6-cyano-2H-1-benzopyran,
trans-6-cyano-2,2-dimethyl-3,4-dihydro-3-(t-butylureido)-2H-1-benzopyran-4-ol,
trans-6-cyano-2,2-dimethyl-3,4-dihydro-3-(3,4-difluorobenzoylamino)-2H-1-benzopyran-4-ol,
trans-6-cyano-2,2-dimethyl-3,4-dihydro-3-(methylureido)-2H-1-benzopyran-4-ol,
trans-6-cyano-2,2-dimethyl-3,4-dihydro-3-acetylamino-2H-1-benzopyran-4-ol,
trans-6-cyano-2,2-dimethyl-3,4-dihydro-3-(2-pyridine-carbonylamino-2H-1-benzopyran-4-ol,
trans-6-cyano-2,2-dimethyl-3,4-dihydro-3-(t-butylacetylamino)-2H-1-benzopyran-4-ol,
trans-6-cyano-2,2-dimethyl-3,4-dihydro-3-(cyclohexane-carbonylamino)-2H-1-benzopyran-4-ol,
trans-6-cyano-2,2-dimethyl-3,4-dihydro-3-(cyclohexylureido)-2H-1-benzopyran-4-ol),
trans-6-cyano-2,2-dimethyl-3,4-dihydro-3-benzoylamino-2H-1-benzopyran-4-ol,
trans-6-cyano-2,2-dimethyl-3,4-dihydro-3-(isopropylureido)-2H-1-benzopyran-4-ol,
trans-6-cyano-2,2-dimethyl-3,4-dihydro-3-(ethylureido)-2H-1-benzopyran-4-ol,
trans-6-cyano-2,2-dimethyl-3,4-dihydro-3-(n-propylureido)-2H-1-benzopyran-4-ol),
trans-6-chloro-2,2-dimethyl-3,4-dihydro-3-(t-butylureido-2H-1-benzopyran-4-ol,
trans-6-cyano-2,2-dimethyl-3,4-dihydro-3-(dimethylaminocarbonylamino)-2H-1-benzopyran-4-ol,
trans-6-cyano-2,2-dimethyl-3,4-dihydro-3-isopropylthioureido-2H-1-benzopyran-4-ol,
trans-6-trifluoromethyl-2,2-dimethyl-3,4-dihydro-3-(t-butylureido-2H-1-benzopyran-4-ol,
trans-6-cyano-2,2-dimethyl-3,4-dihydro-3-trimethylacetylamino-2H-1-benzopyran-4-ol,
trans-6-cyano-2,2-dimethyl-3,4-dihydro-3-isobutyrylamino-2H-1-benzopyran-4-ol,
trans-6-cyano-3,4-dihydro-2,2-dimethyl-3-(benzyloxycarbonyl)amino-2H-1-benzopyran-4-ol,
2,2-dimethyl-3,4-dihydro-3-(t-butylureido)-6-cyano-2H-1-benzopyran,
trans-6-cyano-3,4-dihydro-2,2-dimethyl-3-(t-butoxycarbonyl)amino-4-methoxy-2H-1-benzopyran and
trans-6-cyano-3,4-dihydro-2,2-dimethyl-3-(t-butoxycarbonyl)amino-4-amino-2H-1-benzopyran.

**10.** A process for the preparation of a compound according to claim 1, which process comprises acylating a compound of formula (II):

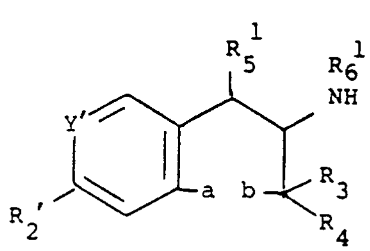

(II)

wherein Y′ and $R_2'$, are Y and $R_2$ respectively or groups or atoms convertible thereto, $R_5^1$ is hydroxy, $C_{1-6}$ alkoxy, $C_{1-7}$ acyloxy or azide, $R_6^1$ is hydrogen or $C_{1-6}$ alkyl and the $R_6^1NH$ group is trans to the $R_5^1$ group (when $R_5^1$ is other than than azide), with

a) an acylating agent of formula (III):

$R_7{}^1COQ$ (III)

wherein Q is a leaving group and $R_7{}^1$ is $R_7$ as defined in claim 1, or a group convertible thereto, when not joined to $R_6$ or, (when $R_6{}^1$ is hydrogen), $R_7{}^1$ is a group $R_{10}$ which is $C_{3-4}$ polymethylene optionally substituted by one or two $C_{1-6}$ alkyl groups and optionally containing one or, (when $C_4$ polymethylene), two, C=C double bond(s), terminally substituted by a leaving group, L; and thereafter, when $R_7{}^1$ is $R_{10}$, cyclising the resulting compound; or

b) (when $R_7$ is $NHR_{11}$ wherein $R_{11}$ is hydrogen or $C_{1-6}$ alkyl), with a compound of formula (IV):

$X = C = NR_{11}$ (IV)

wherein X and $R_{11}$ are as hereinbefore defined;

and thereafter, as desired or necessary, converting $R_5{}^1$ to $R_5$, converting Y′ and/or $R_2'$ to Y and/or $R_2$, optionally thiating X when oxygen to X is sulphur and/or dehydrating a compound wherein $R_5{}^1$ is hydroxy to form a compound of formula (I) wherein $R_5$ and E form a bond or converting a compound of formula (I) wherein the $R_6NCXR_7$ moiety is trans to $R_5$ when hydroxy, to a corresponding cis isomer; and optionally forming a pharmaceutically acceptable salt thereof.

**11.** A compound of formula (II), (V) or (VI):

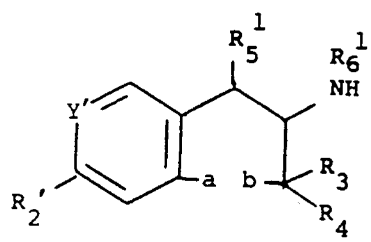

(II)

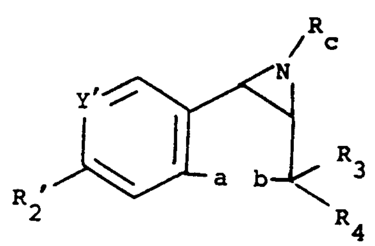

(V)

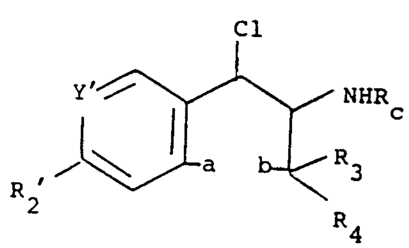

(VI)

wherein $R_c$ is an amino protecting group and the other variable groups are defined in claim 10.

**12.** A compound selected from the group consisting of:
trans-6-cyano-2,2-dimethyl-3,4-dihydro-3-amino-2H-1-benzopyran-4-ol,
trans-6-chloro-2,2-dimethyl-3,4-dihydro-3-amino-2H-1-benzopyran-4-ol,
trans-6-trifluoromethyl-2,2-dimethyl-3,4-dihydro-3-amino-2H-1-benzopyran-4-ol,
6-cyano-3,4-[(N-t-butoxycarbonyl)aziridino]-2,2-dimethyl-2H-1-benzopyran,
6-chloro-3,4-[(N-t-butoxycarbonyl)aziridino]-2,2-dimethyl-2H-1-benzopyran,
6-trifluoromethyl-3,4-[(N-t-butoxycarbonyl)aziridino]-2,2-dimethyl-2H-1-benzopyran,
6-cyano-3,4-[(N-benzyloxycarbonyl)aziridino]- 2,2-dimethyl-2H-1-benzopyran,
trans-6-cyano-3,4-dihydro-2,2-dimethyl-3-(t-butoxycarbonylamino)-4-chloro-2H-1-benzopyran,
trans-6-chloro-3,4-dihydro-2,2-dimethyl-3-(t-butoxycarbonyl)amino-4-chloro-2H-1-benzopyran,
trans-6-trifluoromethyl-3,4-dihydro-2,2-dimethyl-3-(t-butoxycarbonyl)amino-4-chloro-2H-1-benzopyran
and
trans-6-cyano-3,4-dihydro-2,2-dimethyl-3-(benzyloxycarbonylamino)-4-chloro-2H-1-benzopyran.

**13.** A pharmaceutical composition comprising a compound according to any one of claims 1 to 9, and a pharmaceutically acceptable carrier.

**14.** A compound according to any one of claims 1 to 9 for use as an active therapeutic substance.

**15.** A compound according to any one of claims 1 to 9 for use in the treatment of hypertension and/or respiratory tract disorders.

**16.** Use of a compound according to any one of claims 1 to 9 in the manufacture of a medicament for use in the treatment of hypertension and/or respiratory tract disorders.

**Claims for the following Contracting State : ES**

**1.** A process for the preparation of a compound of formula (I), or a pharmaceutically acceptable salt thereof:

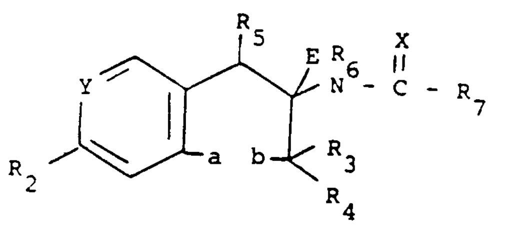

(I)

wherein

a and b together form an -O- linkage or a bond or (when $R_2$ is hydrogen), $CH_2$;
either Y is N and $R_2$ is hydrogen; or
Y is C-$R_1$

wherein

either one of $R_1$ and $R_2$ is hydrogen and the other is nitro, cyano, halo, $CF_3$, $C_2F_5$, formyl, aldoxime, $CF_3O$, $NO_2$-CH=CH-, NC-CH=CH-; a group $R_xX$-wherein $R_x$ is $C_{1-6}$ alkyl, aryl or heteroaryl either of which may be optionally substituted by one, two or three of $C_{1-4}$ alkyl, $C_{1-4}$ alkoxy, nitro, halo, $CF_3$ and cyano; and X is C=O, O.C=O, C=O.O., CHOH, SO, $SO_2$, O.SO, O.$SO_2$, CONH, O.CONH, O.$SO_2$NH, CO-CH=CH, C=NHOH, C=$NNH_2$; or a group $R_yR_zNZ$- wherein $R_y$ and $R_z$ are independently hydrogen or $C_{1-6}$ alkyl and Z is C=O, SO or $SO_2$; or a group $(R_wO)_2P(O)W$ wherein $R_w$ is hydrogen or $C_{1-6}$ alkyl and W is O or a bond; or

$R_1$ is a $C_{3-8}$ cycloalkyl group or a $C_{1-6}$ alkyl group optionally substituted by a group which is hydroxy, $C_{1-6}$ alkoxy, amino optionally substituted by one or two $C_{1-6}$ alkyl groups, $C_{1-7}$ alkanoylamino, $C_{3-8}$ cycloalkyloxy or $C_{3-8}$ cycloalkylamino; and $R_2$ is hydrogen; or

one of $R_1$ and $R_2$ is nitro, cyano or $C_{1-3}$ alkylcarbonyl and the other is a different group selected from nitro, cyano, halo, $C_{1-3}$ alkylcarbonyl, methoxy or amino optionally substituted by one or two

$C_{1-6}$ alkyl or by $C_{2-7}$ alkanoyl; or

$R_1$ and $R_2$ together with the carbon atoms to which they are attached, form 2,1,3-oxadiazole;

either one of $R_3$ and $R_4$ is hydrogen or $C_{1-4}$ alkyl and the other is $C_{1-4}$ alkyl; or $R_3$ and $R_4$ together are $C_{2-5}$ polymethylene

$R_5$ is hydrogen, hydroxy, amino, $C_{1-6}$ alkoxy or $C_{1-7}$ acyloxy; and

E is hydrogen; or

$R_5$ and E together form a bond;

either $R_6$ is hydrogen or $C_{1-6}$ alkyl; and

$R_7$ is hydrogen, $C_{3-8}$ cycloalkyl, $C_{2-6}$ alkenyl, $C_{1-6}$ alkyl optionally substituted by hydroxy, $C_{1-6}$ alkoxy, $C_{1-6}$ alkoxycarbonyl, carboxy, halogen or amino optionally substituted by one or two $C_{1-6}$ alkyl groups; aryl or heteroaryl either being optionally substituted by one or more groups or atoms selected from the class of $C_{1-6}$ alkyl, $C_{1-6}$ alkoxy, hydroxy, halogen, trifluoromethyl, nitro, cyano, $C_{1-12}$ carboxylic acyl, or $R_7$ is amino or aminocarbonyl optionally substituted by one or two $C_{1-6}$ alkyl groups or substituted by $C_{3-8}$ cycloalkyl, or a group $OR_9$ wherein $R_9$ is $C_{1-6}$ alkyl or benzyl optionally substituted as defined for $R_7$; or

$R_6$ and $R_7$ are joined together to form $C_{3-4}$ polymethylene optionally substituted by one or two $C_{1-6}$ alkyl groups and optionally containing one or, (when $C_4$ polymethylene), two, C=C double bond(s); and

X is oxygen or sulphur;

which process comprises acylating a compound of formula (II):

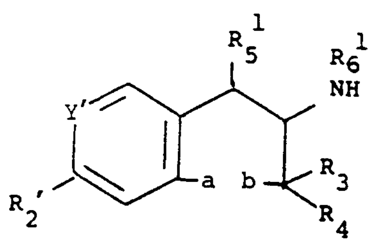

(II)

wherein Y′ and $R_2'$, are Y and $R_2$ respectively or groups or atoms convertible thereto, $R_5{}^1$ is hydroxy, $C_{1-6}$ alkoxy, $C_{1-7}$ acyloxy or azide, $R_6{}^1$ is hydrogen or $C_{1-6}$ alkyl and the $R_6{}^1NH$ group is trans to the $R_5{}^1$ group (when $R_5{}^1$ is other than than azide), with

a) an acylating agent of formula (III):

$R_7{}^1COQ$ (III)

wherein Q is a leaving group and $R_7{}^1$ is $R_7$ as defined, or a group convertible thereto, when not joined to $R_6$ or, (when $R_6{}^1$ is hydrogen), $R_7{}^1$ is a group $R_{10}$ which is $C_{3-4}$ polymethylene optionally substituted by one or two $C_{1-6}$ alkyl groups and optionally containing one or, (when $C_4$ polymethylene), two, C=C double bond(s), terminally substituted by a leaving group, L; and thereafter, when $R_7{}^1$ is $R_{10}$, cyclising the resulting compound; or

b) (when $R_7$ is $NHR_{11}$ wherein $R_{11}$ is hydrogen or $C_{1-6}$ alkyl), with a compound of formula (IV):

$X = C = NR_{11}$ (IV)

wherein X and $R_{11}$ are as hereinbefore defined;

and thereafter, as desired or necessary, converting $R_5{}^1$ to $R_5$, converting Y′ and/or $R_2'$, to Y and/or $R_2$, optionally thiating X when oxygen to X is sulphur and/or dehydrating a compound wherein $R_5{}^1$ is hydroxy to form a compound of formula (I) wherein $R_5$ and E form a bond or converting a compound of formula (I) wherein the $R_6NCXR_7$ moiety is trans to $R_5$ when hydroxy, to a corresponding cis isomer; and optionally forming a pharmaceutically acceptable salt thereof.

**2.** A process according to claim 1 wherein Y is C-$R_1$ and a and b together are an -O- linkage.

**3.** A process according to claim 1 or 2 wherein $R_1$ is nitro, cyano, acetyl, $CF_3$, $C_2F_5$ or $C_{1-4}$ alkyl, and $R_2$ is hydrogen.

**4.** A process according to claim 3 wherein $R_1$ is cyano.

**5.** A process according to any one of claims 1 to 4 wherein $R_3$ and $R_4$ are both methyl groups.

**6.** A process according to any one of claims 1 to 5 wherein $R_5$ is hydroxy and E is hydrogen and the $R_6NCXR_7$ moiety is trans to $R_5$.

**7.** A process according to any one of claims 1 to 6 wherein $R_6$ is hydrogen and $R_7$ is optionally substituted phenyl, branched $C_{1-6}$ alkyl, $C_{1-6}$alkoxy, benzyloxy or optionally substituted amino.

**8.** A process according to claim 7 wherein $R_7$ is t-butylamino.

**9.** A process according to claim 1 for the preparation of a compound selected from the group consisting of:
trans-6-cyano-2,2-dimethyl-3,4-dihydro-3-(t-butoxycarbonyl)amino-2H-1-benzopyran-4-ol,
trans-6-cyano-2,2-dimethyl-3,4-dihydro-3-(2-oxo-1-piperidinyl)-2H-1-benzopyran-4-ol,
trans-6-cyano-2,2-dimethyl-3,4-dihydro-3-(4-fluorobenzoylamino)-2H-1-benzopyran-4-ol,
2,2-dimethyl-3,4-dihydro-3-(t-butoxycarbonyl)amino-6-cyano-2H-1-benzopyran,
trans-6-cyano-2,2-dimethyl-3,4-dihydro-3-(t-butylureido)-2H-1-benzopyran-4-ol,
trans-6-cyano-2,2-dimethyl-3,4-dihydro-3-(3,4-difluorobenzoylamino-2H-1-benzopyran-4-ol,
trans-6-cyano-2,2-dimethyl-3,4-dihydro-3-(methylureido-2H-1-benzopyran-4-ol,
trans-6-cyano-2,2-dimethyl-3,4-dihydro-3-acetylamino-2H-1-benzopyran-4-ol,
trans-6-cyano-2,2-dimethyl-3,4-dihydro-3-(2-pyridine-carbonylamino)-2H-1-benzopyran-4-ol,
trans-6-cyano-2,2-dimethyl-3,4-dihydro-3-(t-butylacetylamino)-2H-1-benzopyran-4-ol,
trans-6-cyano-2,2-dimethyl-3,4-dihydro-3-(cyclohexane-carbonylamino-2H-1-benzopyran-4-ol,
trans-6-cyano-2,2-dimethyl-3,4-dihydro-3-(cyclohexylureido)-2H-1-benzopyran-4-ol),
trans-6-cyano-2,2-dimethyl-3,4-dihydro-3-benzoylamino-2H-1-benzopyran-4-ol,
trans-6-cyano-2,2-dimethyl-3,4-dihydro-3-(isopropylureido)-2H-1-benzopyran-4-ol,
trans-6-cyano-2,2-dimethyl-3,4-dihydro-3-(ethylureido)-2H-1-benzopyran-4-ol,
trans-6-cyano-2,2-dimethyl-3,4-dihydro-3-(n-propylureido)-2H-1-benzopyran-4-ol),
trans-6-chloro-2,2-dimethyl-3,4-dihydro-3-(t-butylureido)-2H-1-benzopyran-4-ol,
trans-6-cyano-2,2-dimethyl-3,4-dihydro-3-(dimethylaminocarbonylamino)-2H-1-benzopyran-4-ol,
trans-6-cyano-2,2-dimethyl-3,4-dihydro-3-(isopropylthioureido)-2H-1-benzopyran-4-ol,
trans-6-trifluoromethyl-2,2-dimethyl-3,4-dihydro-3-(t-butylureido)-2H-1-benzopyran-4-ol,
trans-6-cyano-2,2-dimethyl-3,4-dihydro-3-trimethylacetylamino-2H-1-benzopyran-4-ol,
trans-6-cyano-2,2-dimethyl-3,4-dihydro-3-isobutyrylamino-2H-1-benzopyran-4-ol,
trans-6-cyano-3,4-dihydro-2,2-dimethyl-3-(benzyloxycarbonyl)amino-2H-1-benzopyran-4-ol,
2,2-dimethyl-3,4-dihydro-3-(t-butylureido)-6-cyano-2H-1-benzopyran,
trans-6-cyano-3,4-dihydro-2,2-dimethyl-3-(t-butoxycarbonyl)amino-4-methoxy-2H-1-benzopyran and
trans-6-cyano-3,4-dihydro-2,2-dimethyl-3-(t-butoxycarbonyl)amino-4-amino-2H-1-benzopyran.

**10.** Use of a compound of formula (I) as defined in any one of claims 1 to 9, or a pharmaceutically acceptable salt thereof, in the manufacture of a medicament for use in the treatment of hypertension and/or respiratory tract disorders.

## Patentansprüche

## Patentansprüche für folgende Vertragsstaaten : AT, BE, CH, DE, FR, GB, GR, IT, LI, LU, NL, SE

**1.** Verbindung der Formel (I) oder ein pharmazeutisch verträgliches Salz davon

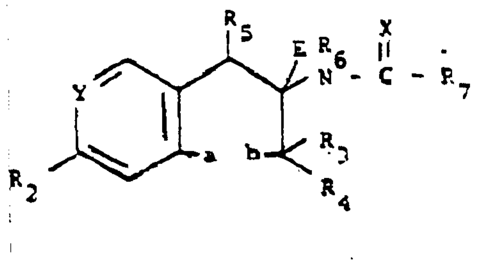

(I)

wobei

a und b gemeinsam eine -O-Brücke oder eine Bindung oder, wenn $R_2$ ein Wasserstoffatom ist, eine $CH_2$-Gruppe bilden;

entweder Y ein Stickstoffatom und $R_2$ ein Wasserstoffatom ist; oder

Y ein C-$R_1$-Rest ist,

wobei

entweder einer der Reste $R_1$ und $R_2$ ein Wasserstoffatom ist und der andere eine Nitro- oder Cyanogruppe, ein Halogenatom oder die Gruppe $CF_3$, $C_2F_5$, Formyl, Aldoxim, $CF_3O$, $NO_2CH=CH$ oder NC-CH=CH ist; oder einen Rest $R_xX$ bedeutet, wobei $R_x$ einen $C_{1-6}$-Alkyl, Aryl- oder Heteroarylrest darstellt, gegebenenfalls substituiert durch einen, zwei oder drei $C_{1-4}$-Alkyl- oder $C_{1-4}$-Alkoxyreste, Nitrogruppen, Halogenatome, $CF_3$- oder Cyanogruppen; und X die Gruppe C=O, O-C=O, (C=O)O, CHOH, SO, $SO_2$, O-SO, O-$SO_2$, CONH, O-CONH, O-$SO_2$NH, CO-CH=CH, C=NHOH oder C=$NNH_2$ bedeutet, oder ein Rest $R_yR_zNZ$ ist, wobei $R_y$ und $R_z$ unabhängig voneinander ein Wasserstoffatom oder ein $C_{1-6}$-Alkylrest sind und Z die Gruppe C=O, SO oder $SO_2$ darstellt; oder ein Rest $(R_wO)_2P(O)$-W ist, wobei $R_w$ ein Wasserstoffatom oder ein $C_{1-6}$-Alkylrest ist und W ein Sauerstoffatom oder eine Bindung bedeutet; oder

$R_1$ ein $C_{3-8}$-Cycloalkyl- oder $C_{1-6}$-Alkylrest ist, welcher gegebenenfalls durch eine Hydroxygruppe, einen $C_{1-6}$-Alkoxyrest, einen Aminorest, der gegebenenfalls durch einen oder zwei $C_{1-6}$-Alkylreste substituiert ist, einen $C_{1-7}$-Alkanoylamino-, $C_{3-8}$-Cycloalkyloxy- oder $C_{3-8}$-Cyoloalkylaminorest substituiert ist; und $R_2$ ein Wasserstoffatom ist; oder

einer der Reste $R_1$ und $R_2$ eine Nitro- oder Cyanogruppe oder einen $C_{1-3}$-Alkylcarbonylrest darstellt und der andere ein davon verschiedener Rest ist, gewählt aus einer Nitro- oder Cyanogruppe, einem Halogenatom, einem $C_{1-3}$-Alkylcarbonylrest, einer Methoxygruppe oder einem Aminorest, der gegebenenfalls durch einen oder zwei $C_{1-6}$-Alkyl- oder durch $C_{2-7}$-Alkanoylrestesubstituiert ist; oder

$R_1$ und $R_2$ gemeinsam mit den Kohlenstoffatomen, an welche sie gebunden sind, ein 2,1,3-Oxadiazol bilden;

einer der Reste $R_3$ und $R_4$ ein Wasserstoffatom oder ein $C_{1-4}$-Alkylrest und der andere ein $C_{1-4}$-Alkylrest ist; oder $R_3$ und $R_4$ gemeinsam einen $C_{2-5}$-Polymethylenrest bedeuten;

$R_5$ ein Wasserstoffatom, eine Hydroxygruppe, ein Amino-, $C_{1-6}$-Alkoxy- oder $C_{1-7}$-Acyloxyrest ist; und

E ein Wasserstoffatom ist; oder

$R_5$ und E gemeinsam eine Bindung bilden;

$R_6$ entweder ein Wasserstoffatom oder einen $C_{1-6}$-Alkylrest darstellt; und

$R_7$ ein Wasserstoffatom, einen $C_{3-8}$-Cycloalkyl, einen $C_{2-6}$-Alkenyl-, einen gegebenenfalls durch eine Hydroxygruppe substituierten $C_{1-6}$-Alkylrest, einen $C_{1-6}$-Alkoxy-,einen $C_{1-6}$-Alkoxycarbonylrest, eine Carbonsäuregruppe, ein Halogenatom oder einen gegebenenfalls durch einen oder zwei $C_{1-6}$-Alkylreste substituierten Aminorest; einen Aryl- oder Heteroarylrest, gegebenenfalls substituiert durch einen oder mehrere Reste oder Atome, gewählt aus einem $C_{1-6}$-Alkyl- oder $C_{1-6}$-Alkoxylrest, der Hydroxygruppe, einem Halogenatom, der Trifluormethyl-, Nitro- oder Cyanogruppe, einem $C_{1-12}$-Carboxylacylrest, oder $R_7$ einen Amino- oder Aminocarbonylrest bedeutet, der gegebenenfalls durch einen oder zwei $C_{1-6}$-Alkylreste oder durch einen $C_{3-8}$-Cycloalkylrest substituiert ist, oder ein Rest $OR_9$ ist, bei dem $R_9$ einen $C_{1-6}$-Alkyl- oder Benzylrest bedeutet, der gegebenenfalls, wie bei $R_7$ definiert, substituiert ist; oder

$R_6$ und $R_7$ miteinander unter Bildung eines $C_{3-4}$-Polymethylenrests verbunden sind, der gegebenenfalls durch einen oder zwei $C_{1-6}$-Alkylreste substituiert ist und gegebenenfalls eine oder (wenn es sich um einen $C_4$-Polymethylenrest handelt) zwei C=C-Doppelbindungen enthält; und
X ein Sauerstoff- oder Schwefelatom ist.

**2.** Verbindung nach Anspruch 1, wobei Y ein C-$R_1$-Rest ist und a und b gemeinsam eine -O-Brücke bilden.

**3.** Verbindung nach Anspruch 1 oder 2, wobei $R_1$ eine Nitro-, eine Cyano-, eine Acetyl-, eine $CF_3$-, eine $C_2F_5$-Gruppe oder einen $C_{1-4}$-Alkylrest bedeutet und $R_2$ ein Wasserstoffatom ist.

**4.** Verbindung nach Anspruch 3, wobei $R_1$ eine Cyanogruppe ist.

**5.** Verbindung nach einem der Ansprüche 1 bis 4, wobei $R_3$ und $R_4$ jeweils eine Methylgruppe sind.

**6.** Verbindung nach einem der Ansprüche 1 bis 5, wobei $R_5$ eine Hydroxygruppe ist und E ein Wasserstoffatom darstellt und die $R_6NCXR_7$-Einheit trans-ständig zu $R_5$ ist.

**7.** Verbindung nach einem der Ansprüche 1 bis 6, wobei $R_6$ ein Wasserstoffatom ist und $R_7$ einen gegebenenfalls substituierten Phenyl-, einen verzweigten $C_{1-6}$-Alkyl-, einen $C_{1-6}$-Alkoxy-, einen Benzyloxyrest oder einen gegebenenfalls substituierten Aminorest bedeutet.

**8.** Verbindung nach Anspruch 7, wobei $R_7$ eine tert-Butylaminogruppe ist.

**9.** Verbindung, gewählt aus der Gruppe, bestehend aus:
trans-6-Cyano-2,2-dimethyl-3,4-dihydro-3-(t-butoxycarbonyl)amino-2H-1-benzopyran-4-ol,
trans-6-Cyano-2,2-dimethyl-3,4-dihydro-3-(2-oxo-1-piperidinyl)-2H-1-benzopyran-4-ol,
trans-6-Cyano-2,2-dimethyl-3,4-dihydro-3-(4-fluorobenzoylamino)-2H-1-benzopyran-4-ol,
2,2-Dimethyl-3,4-dihydro-3-(t-butoxycarbonyl)amino-6-cyano-2H-1-benzopyran,
trans-6-Cyano-2,2-dimethyl-3,4-dihydro-3-(t-butylureido)-2H-1-benzopyran-4-ol,
trans-6-Cyano-2,2-dimethyl-3,4-dihydro-3-(3,4-difluorobenzoylamino)-2H-1-benzopyran-4-ol,
trans-6-Cyano-2,2-dimethyl-3,4-dihydro-3-(methylureido)-2H-1-benzopyran-4-ol,
trans-6-Cyano-2,3-dimethyl-3,4-dihydro-3-acetylamino-2H-1-benzopyran-4-ol,
trans-6-Cyano-2,2-dimethyl-3,4-dihydro-3-(2-pyridincarbonylamino)-2H-1-benzopyran-4-ol,
trans-6-Cyano-2,2-dimethyl-3,4-dihydro-3-(t-butylacetylamino)-2H-1-benzopyran-4-ol,
trans-6-Cyano-2,2-dimethyl-3,4-dihydro-3-(cyclohexancarbonylamino)-2H-1-benzopyran-4-ol,
trans-6-Cyano-2,2-dimethyl-3,4-dihydro-3-(cyclohexylureido)-2H-1-benzopyran-4-ol,
trans-6-Cyano-2,2-dimethyl-3,4-dihydro-3-benzoylamino-2H-1-benzopyran-4-ol,
trans-6-Cyano-2,2-dimethyl-3,4-dihydro-3-(isopropylureido)-2H-1-benzopyran-4-ol,
trans-6-Cyano-2,2-dimethyl-3,4-dihydro-3-(ethylureido)-2H-1-benzopyran-4-ol,
trans-6-Cyano-2,2-dimethyl-3,4-dihydro-3-(n-propylureido)-2H-1-benzopyran-4-ol,
trans-6-Chloro-2,2-dimethyl-3,4-dihydro-3-(t-butylureido)-2H-1-benzopyran-4-ol,
trans-6-Cyano-2,2-dimethyl-3,4-dihydro-3-(dimethylaminocarbonylamino)-2H-1-benzopyran-4-ol,
trans-6-Cyano-2,2-dimethyl-3,4-dihydro-3-(isopropylthioureido)-2H-1-benzopyran-4-ol,
trans-6-Trifluoromethyl-2,2-dimethyl-3,4-dihydro-3-(t-butylureido)-2H-1-benzopyran-4-ol,
trans-6-Cyano-2,2-dimethyl-3,4-dihydro-3-trimethylacetylamino-2H-1-benzopyran-4-ol,
trans-6-Cyano-2,2-dimethyl-3,4-dihydro-3-isobutyrylamino-2H-1-benzopyran-4-ol,
trans-6-Cyano-3,4-dihydro-2,2-dimethyl-3-(benzyloxy-carbonyl)amino-2H-1-benzopyran-4-ol,
2,2-Dimethyl-3,4-dihydro-3-(t-butylureido)-6-cyano-2H-1-benzopyran,
trans-6-Cyano-3,4-dihydro-2,2-dimethyl-3-(t-butoxycarbonyl)amino-4-methoxy-2H-1-benzopyran und
trans-6-Cyano-3,4-dihydro-2,2-dimethyl-3-(t-butoxycarbonyl)amino-4-amino-2H-1-benzopyran.

**10.** Verfahren zur Herstellung einer Verbindung nach Anspruch 1, wobei das Verfahren umfaßt: Acylieren einer Verbindung der Formel (II)

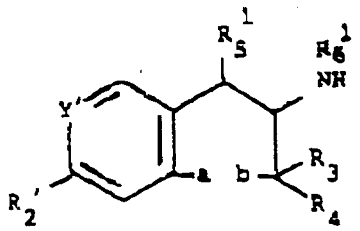

(II)

wobei Y' und $R_2$' jeweils Y und $R_2$ oder Reste oder Atome sind, die dazu umgewandelt werden können, $R_5{}^1$ eine Hydroxygruppe, ein $C_{1-6}$-Alxoxy-, ein $C_{1-7}$-Acyloxy- oder ein Azidrest ist, $R_6{}^1$ ein Wasserstoffatom oder einen $C_{1-6}$-Alkylrest bedeutet und der $R_6{}^1$NH-Rest zum $R_5{}^1$-Rest (wenn $R_5{}^1$ etwas anderes als der Azidrest ist) transständig ist, mit

a) einem Acylierungsmittel der Formel (III)

$R_7{}^1$COQ (III),

wobei Q eine Austrittsgruppe ist und $R_7{}^1$ ein $R_7$-Rest, wie in Anspruch 1 definiert, ist oder einen Rest darstellt, der dazu, wenn er nicht an $R_6$ gebunden ist, umgewandelt werden kann, oder (wenn $R_6{}^1$ ein Wasserstoffatom ist) $R_7{}^1$ einen $R_{10}$-Rest darstellt, der einen $C_{3-4}$-Polymethylenrest bedeutet, welcher gegebenenfalls mit einem oder zwei $C_{1-6}$-Alkylresten substituiert ist und gegebenenfalls eine oder (wenn es sich um einen $C_4$-Polymethylenrest handelt) zwei C = C-Doppelbindungen enthält, welche(r) terminal durch eine Austrittsgruppe L substituiert (ist) sind; und danach, wenn $R_7{}^1$ für $R_{10}$ steht, die gebildete Verbindung cyclisiert wird; oder

b) (wenn $R_7$ ein $NHR_{11}$-Rest ist, wobei $R_{11}$ ein Wasserstoffatom oder ein $C_{1-6}$-Alkylrest ist) mit einer Verbindung der Formel (IV)

$X = C = NR_{11}$ (IV),

wobei X und $R_{11}$ wie vorstehend definiert sind;

und danach, wie gewünscht oder notwendig, Umwandeln von $R_5{}^1$ in $R_5$, Umwandeln von Y' und/oder $R_2$' in Y und/oder $R_2$, wahlweise Thiierung, wenn X ein Sauerstoffatom ist, zu einer Verbindung, in der X ein Schwefelatom ist, und/oder Dehydrieren einer Verbindung, bei welcher $R_5{}^1$ eine Hydroxygruppe ist, um eine Verbindung dir Formel (I) zu bilden, bei der $R_5$ und E eine Bindung bilden oder Umwandeln einer Verbindung der Formel (I), bei der, wenn $R_5$ eine Hydroxygruppe ist, die $R_6NHXR_7$-Einheit trans-ständig zu $R_5$ ist, zu einem entsprechenden cis-Isomeren;
und gegebenenfalls Bilden eines pharmazeutisch verträglichen Salzes davon.

**11.** Verbindung der Formel (II), (V) oder (VI):

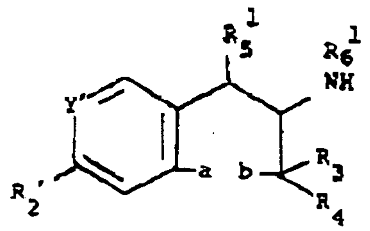

(II)

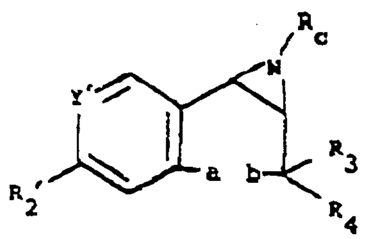

(V)

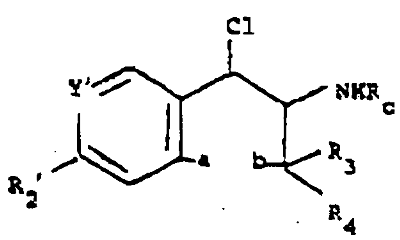

(VI)

wobei $R_c$ eine Amino-Schutzgruppe ist und die anderen variablen Reste wie in Anspruch 10 definiert sind.

**12.** Verbindung, gewählt aus der Gruppe, bestehend aus:
trans-6-Cyano-2,2-dimethyl-3,4-dihydro-3-amino-2H-1-benzopyran-4-ol,
trans-6-Chloro-2,2-dimethyl-3,4-dihydro-3-amino-2H-1-benzopyran-4-ol,
trans-6-Trifluoromethyl-2,2-dimethyl-3,4-dihydro-3-amino-2H-1-benzopyran-4-ol,
6-Cyano-3,4-[(N-t-butoxycarbonyl)aziridino]-2,2-dimethyl-2H-1-benzopyran,
6-Chloro-3,4-[(N-t-butoxycarbonyl)aziridino]-2,2-dimethyl-2H-1-benzopyran,
6-Trifluoromethyl-3,4-[(N-t-butoxycarbonyl)aziridino]-2,2-dimethyl-2H-1-benzopyran,
6-Cyano-3,4-[(N-benzyloxycarbonyl)aziridino]-2,2-dimethyl-2H-1-benzopyran,
trans-6-Cyano-3,4-dihydro-2,2-dimethyl-3-(t-butoxycarbonylamino)-4-chloro-2H-1-benzopyran,
trans-6-Chloro-3,4-dihydro-2,2-dimethyl-3-(t-butoxycarbonyl)amino-4-chloro-2H-1-benzopyran,
trans-6-Trifluoromethyl-3,4-dihydro-2,2-dimethyl-3-(t-butoxycarbonyl)amino-4-chloro-2H-1-benzopyran und
trans-6-Cyano-3,4-dihydro-2,2-dimethyl-3-(benzyloxycarbonylamino)-4-chloro-2H-1-benzopyran.

**13.** Arzneimittel umfassend eine Verbindung nach einem der Ansprüche 1 bis 9 und eine pharmazeutisch verträgliche Trägersubstanz.

**14.** Verbindung nach einem der Ansprüche 1 bis 9 zur Verwendung als wirksame, therapeutische Substanz.

15. Verbindung nach einem der Ansprüche 1 bis 9 zur Verwendung bei der Behandlung von Hypertonie und/oder Atemwegestörungen.

16. Verwendung einer Verbindung nach einem der Ansprüche 1 bis 9 für die Herstellung eines Medikaments zur Verwendung bei der Behandlung von Hypertonie und/oder Atemwegsstörungen.

**Patentansprüche für folgenden Vertragsstaat : ES**

1. Verfahren zur Herstellung einer Verbindung der Formel (I) oder eines pharmazeutisch verträglichen Salzes davon

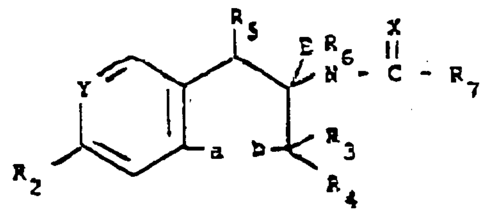

(I)

wobei

a und b gemeinsam eine -O-Brücke oder eine Bindung oder, wenn $R_2$ ein Wasserstoffatom ist, eine $CH_2$-Gruppe bilden;

entweder Y ein Stickstoffatom und $R_2$ ein Wasserstoffatom ist; oder

Y ein C-$R_1$-Rest ist,

wobei

entweder einer der Reste $R_1$ und $R_2$ ein Wasserstoffatom ist und der andere eine Nitro- oder Cyanogruppe, ein Halogenatom oder die Gruppe $CF_3$, $C_2F_5$, Formyl, Aldoxim, $CF_3O$, $NO_2CH=CH$ oder NC-CH=CH ist; oder einen Rest $R_xX$ bedeutet, wobei $R_x$ einen $C_{1-6}$-Alkyl, Aryl- oder Heteroarylrest darstellt, gegebenenfalls substituiert durch einen, zwei oder drei $C_{1-4}$-Alkyl- oder $C_{1-4}$-Alkoxyreste, Nitrogruppen, Halogenatoms, $CF_3$- oder Cyanogruppen; und X die Gruppe C=O, O-C=O, (C=O)O, CHOH, SO, $SO_2$, O-SO, O-$SO_2$, CONH, O-CONH, O-$SO_2$NH, CO-CH=CH, C=NHOH oder C=$NNH_2$ bedeutet, oder ein Rest $R_yR_zNZ$ ist, wobei $R_y$ und $R_z$ unabhängig voneinander ein Wasserstoffatom oder ein $C_{1-6}$-Alkylrest sind und Z die Gruppe C=O, SO oder $SO_2$ darstellt; oder ein Rest $(R_wO)_2P(O)$-W ist, wobei $R_w$ ein Wasserstoffatom oder ein $C_{1-6}$-Alkylrest ist und W ein Sauerstoffatom oder eine Bindung bedeutet; oder

$R_1$ ein $C_{3-8}$-Cycloalkyl- oder $C_{1-6}$-Alkylrest ist, welcher gegebenenfalls durch eine Hydroxygruppe, einen $C_{1-6}$-Alkoxyrest, einen Aminorest, der gegebenenfalls durch einen oder zwei $C_{1-6}$-Alkylreste substituiert ist, einen $C_{1-7}$-Alkanoylamino-, $C_{3-8}$-Cycloalkyloxy- oder $C_{3-8}$-Cycloalkylaminorest substituiert ist; und $R_2$ ein Wasserstoffatom ist; oder

einer der Reste $R_1$ und $R_2$ eine Nitro- oder Cyanogruppe oder einen $C_{1-3}$-Alkylcarbonylrest darstellt und der andere ein davon verschiedener Rest ist, gewählt aus einer Nitro- oder Cyanogruppe, einem Halogenatom, einem $C_{1-3}$-Alkylcarbonylrest, einer Methoxygruppe oder einem Aminorest, der gegebenenfalls durch einen oder zwei $C_{1-6}$-Alkyl- oder durch $C_{2-7}$-Alkanoylrestesubstituiert ist; oder

$R_1$ und $R_2$ gemeinsam mit den Kohlenstoffatomen, an welche sie gebunden sind, ein 2,1,3-Oxadiazol bilden;

einer der Reste $R_3$ und $R_4$ ein Wasserstoffatom oder ein $C_{1-4}$-Alkylrest und der andere ein $C_{1-4}$-Alkylrest ist; oder $R_3$ und $R_4$ gemeinsam einen $C_{2-5}$-Polymethylenrest bedeuten;

$R_5$ ein Wasserstoffatom, eine Hydroxygruppe, ein Amino-, $C_{1-6}$-Alkoxy- oder $C_{1-7}$-Acyloxyrest ist; und

E ein Wasserstoffatom ist; oder

$R_5$ und E gemeinsam eine Bindung bilden;

$R_6$ entweder ein Wasserstoffatom oder einen $C_{1-6}$-Alkylrest darstellt; und

$R_7$ ein Wasserstoffatom, einen $C_{3-8}$-Cycloalkyl, einen $C_{2-6}$-Alkenyl-, einen gegebenenfalls durch eine Hydroxygruppe substituierten $C_{1-6}$-Alkylrest, einen $C_{1-6}$-Alkoxy-,einen $C_{1-6}$-Alkoxycarbonylrest, eine Carbonsäuregruppe, ein Halogenatom oder einen gegebenenfalls durch einen oder zwei $C_{1-6}$-Alkylreste substituierten Aminorest; einen Aryl- oder Heteroarylrest, gegebenenfalls substituiert durch einen oder mehrere Reste oder Atome, gewählt aus einem $C_{1-6}$-Alkyl- oder $C_{1-6}$-Alkoxylrest, der

Hydroxygruppe, einem Halogenatom, der Trifluormethyl-, Nitro- oder Cyanogruppe, einem $C_{1-12}$-Carboxylacylrest, oder $R_7$ einen Amino- oder Aminocarbonylrest bedeutet, der gegebenenfalls durch einen oder zwei $C_{1-6}$-Alkylreste oder durch einem $C_{3-8}$-Cycloalkylrest substituiert ist, oder ein Rest $OR_9$ ist, bei dem $R_9$ einen $C_{1-6}$-Alkyl- oder Benzylrest bedeutet, der gegebenenfalls, wie bei $R_7$ definiert, substituiert ist; oder

$R_6$ und $R_7$ miteinander unter Bildung eines $C_{3-4}$-Polymethylenrests verbunden sind, der gegebenenfalls durch einen oder zwei $C_{1-6}$-Alkylreste substituiert ist und gegebenenfalls eine oder (wenn es sich um einen $C_4$-Polymethylenrest handelt) zwei C = C-Doppelbindungen enthält; und

X ein Sauerstoff- oder Schwefelatom ist,

wobei das Verfahren umfaßt: Acylieren einer Verbindung der Formel (II)

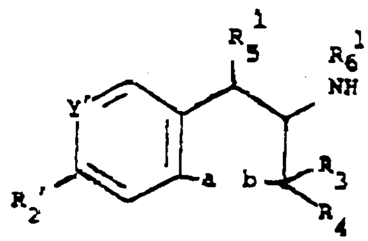

(II)

wobei Y' und $R_2$' jeweils Y und $R_2$ oder Reste oder Atome sind, die dazu umgewandelt werden können, $R_5{}^1$ eine Hydroxygruppe, ein $C_{1-6}$-Alkoxy-, ein $C_{1-7}$-Acyloxy- oder ein Azidrest ist, $R_6{}^1$ ein Wasserstoffatom oder einen $C_{1-6}$-Alkylrest bedeutet und der $R_6{}^1$NH-Rest zum $R_5{}^1$-Rest (wenn $R_5{}^1$ etwas anderes als der Azidrest ist) transständig ist, mit

a) einem Acylierungsmittel der Formel (III)

$R_7{}^1$COQ (III),

wobei Q eins Austrittsgruppe ist und $R_7{}^1$ ein $R_7$-Rest, wie in Anspruch 1 definiert, ist oder einen Rest darstellt, der dazu, wenn er nicht an $R_6$ gebunden ist, umgewandelt werden kann, oder (wenn $R_6{}^1$ ein Wasserstoffatom ist) $R_7{}^1$ einen $R_{10}$-Rest darstellt, der einen $C_{3-4}$-Polymethylenrest bedeutet, welcher gegebenenfalls mit einem oder zwei $C_{1-6}$-Alkylresten substituiert ist und gegebenenfalls eine oder (wenn es sich um einen $C_4$-Polymethylenrest handelt) zwei C = C-Doppelbindungen enthält, welche(r) terminal durch eine Austrittsgruppe L substituiert (ist) sind; und danach, wenn $R_7{}^1$ für $R_{10}$ steht, die gebildete Verbindung cyclisiert wird; oder

b) (wenn $R_7$ ein $NHR_{11}$-Rest ist, wobei $R_{11}$ ein Wasserstoffatom oder ein $C_{1-6}$-Alkylrest ist) mit einer Verbindung der Formel (IV)

$X = C = NR_{11}$ (IV),

wobei X und $R_{11}$ wie vorstehend definiert sind;

und danach, wie gewünscht oder notwendig, Umwandeln von $R_5{}^1$ in $R_5$, Umwandeln von Y' und/oder $R_2$' in Y und/oder $R_2$, wahlweise Thiierung, wenn X ein Sauerstoffatom ist, zu einer Verbindung, in der X ein Schwefelatom ist, und/oder Dehydrieren einer Verbindung, bei welcher $R_5{}^1$ eine Hydroxygruppe ist, um eine Verbindung der Formel (I) zu bilden, bei der $R_5$ und E eine Bindung bilden oder Umwandeln einer Verbindung der Formel (I); bei der, wenn $R_5$ eine Hydroxygruppe ist, die $R_6NHXR_7$-Einheit trans-ständig zu $R_5$ ist, zu einem entsprechenden cis-Isomeren;

und gegebenenfalls Bilden eines pharmazeutisch verträglichen Salzes davon.

**2.** Verfahren nach Anspruch 1, wobei Y ein C-$R_1$-Rest ist und a und b gemeinsam eine -O-Brücke bilden.

**3.** Verfahren nach Anspruch 1 oder 2, wobei $R_1$ eine Nitro-, eine Cyano-, eine Acetyl-, eine $CF_3$-, eine $C_2F_5$-Gruppe oder einen $C_{1-4}$-Alkylrest bedeutet und $R_2$ ein Wasserstoffatom ist.

**4.** Verfahren nach Anspruch 3, wobei $R_1$ eine Cyanogruppe ist.

5. Verfahren nach einem der Ansprüche 1 bis 4, wobei $R_3$ und $R_4$ jeweils eine Methylgruppe sind.

6. Verfahren nach einem der Ansprüche 1 bis 5, wobei $R_5$ eine Hydroxygruppe ist und E ein Wasserstoffatom darstellt und die $R_6NCXR_7$-Einheit trans-ständig zu $R_5$ ist.

7. Verfahren nach einem der Ansprüche 1 bis 6, wobei $R_6$ ein Wasserstoffatom ist und $R_7$ einen gegebenenfalls substituierten Phenyl-, einen verzweigten $C_{1-6}$-Alkyl-, einen $C_{1-6}$-Alkoxy-, einen Benzyloxyrest oder einen gegebenenfalls substituierten Aminorest bedeutet.

8. Verfahren nach Anspruch 7, wobei $R_7$ eine tert-Butylaminogruppe ist.

9. Verfahren nach Anspruch 1 zur Herstellung einer Verbindung, die gewählt wird aus der Gruppe bestehend aus:
trans-6-Cyano-2,2-dimethyl-3,4-dihydro-3-(t-butoxycarbonyl)amino-2H-1-benzopyran-4-ol,
trans-6-Cyano-2,2-dimethyl-3,4-dihydro-3-(2-oxo-1-piperidinyl)-2H-1-benzopyran-4-ol,
trans-6-Cyano-2,2-dimethyl-3,4-dihydro-3-(4-fluorobenzoylamino)-2H-1-benzopyran-4-ol,
2,2-Dimethyl-3,4-dihydro-3-(t-butoxycarbonyl)amino-6-cyano-2H-1-benzopyran,
trans-6-Cyano-2,2-dimethyl-3,4-dihydro-3-(t-butylureido)-2H-1-benzopyran-4-ol,
trans-6-Cyano-2,2-dimethyl-3,4-dihydro-3-(3,4-difluorobenzoylamino)-2H-1-benzopyran-4-ol,
trans-6-Cyano-2,2-dimethyl-3,4-dihydro-3-(methylureido)-2H-1-benzopyran-4-ol,
trans-6-Cyano-2,2-dimethyl-3,4-dihydro-3-acetylamino-2H-1-benzopyran-4-ol,
trans-6-Cyano-2,2-dimethyl-3,4-dihydro-3-(2-pyridincarbonylamino)-2H-1-benzopyran-4-ol,
trans-6-Cyano-2,2-dimethyl-3,4-dihydro-3-(t-butylacetylamino)-2H-1-benzopyran-4-ol,
trans-6-Cyano-2,2-dimethyl-3,4-dihydro-3-(cyclohexancarbonylamino)-2H-1-benzopyran-4-ol,
trans-6-Cyano-2,2-dimethyl-3,4-dihydro-3-(cyclohexylureido)-2H-1-benzopyran-4-ol,
trans-6-Cyano-2,2-dimethyl-3,4-dihydro-3-benzoylamino-2H-1-benzopyran-4-ol,
trans-6-Cyano-2,2-dimethyl-3,4-dihydro-3-(isopropylureido)-2H-1-benzopyran-4-ol,
trans-6-Cyano-2,2-dimethyl-3,4-dihydro-3-(ethylureido)-2H-1-benzopyran-4-ol,
trans-6-Cyano-2,2-dimethyl-3,4-dihydro-3-(n-propylureido)-2H-1-benzopyran-4-ol,
trans-6-Chloro-2,2-dimethyl-3,4-dihydro-3-(t-butylureido)-2H-1-benzopyran-4-ol,
trans-6-Cyano-2,2-dimethyl-3,4-dihydro-3-(dimethylaminocarbonylamino)-2H-1-benzopyran-4-ol,
trans-6-Cyano-2,2-dimethyl-3,4-dihydro-3-(isopropylthioureido)-2H-1-benzopyran-4-ol,
trans-6-Trifluoromethyl-2,2-dimethyl-3,4-dihydro-3-(t-butylureido)-2H-1-benzopyran-4-ol,
trans-6-Cyano-2,2-dimethyl-3,4-dihydro-3-trimethylacetylamino-2H-1-benzopyran-4-ol,
trans-6-Cyano-2,2-dimethyl-3,4-dihydro-3-isobutyrylamino-2H-1-benzopyran-4-ol,
trans-6-Cyano-3,4-dihydro-2,2-dimethyl-3-(benzyloxy-carbonyl)amino-2H-1-benzopyran-4-ol,
2,2-Dimethyl-3,4-dihydro-3-(t-butylureido)-6-cyano-2H-1-benzopyran,
trans-6-Cyano-3,4-dihydro-2,2-dimethyl-3-(t-butoxycarbonyl)amino-4-methoxy-2H-1-benzopyran und
trans-6-Cyano-3,4-dihydro-2,2-dimethyl-3-(t-butoxycarbonyl)amino-4-amino-2H-1-benzopyran.

10. Verwendung einer Verbindung der Formel (I), wie in einem der Ansprüche 1 bis 9 definiert, oder eines pharmazeutisch verträglichen Salzes davon, für die Herstellung eines Medikaments zur Verwendung bei der Behandlung von Hypertonie und/oder Atemwegsstörungen.

**Revendications**
**Revendications pour les Etats contractants suivants : AT, BE, CH, DE, FR, GB, GR, IT, LI, LU, NL, SE**

1. Composé de formule (I) ou sel de celui-ci acceptable du point de vue pharmaceutique:

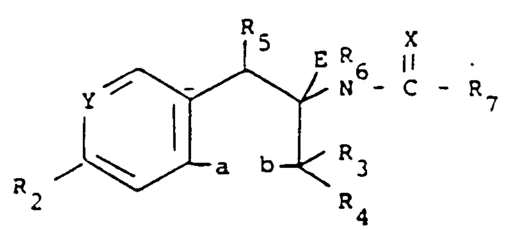

(I)

dans laquelle :

a et b forment ensemble une liaison -O- ou une liaison, ou bien un radical $CH_2$ lorsque $R_2$ est un atome d'hydrogène;

soit Y est un atome d'azote et $R_2$ est un atome d'hydrogène;

soit Y est un radical C-$R_1$

où

l'un ou l'autre des $R_1$ et $R_2$ est un atome d'hydrogène et l'autre est un groupe nitro, cyano, un atome d'halogène, un groupe $CF_3$, $C_2F_5$, formyle, aldoxime, $CF_3O$, $NO_2$-CH=CH-, NC-CH=CH-; un groupe $R_xX$- dans lequel $R_x$ est un groupe alkyle en $C_{1-6}$, aryle ou hétéroaryle, l'un ou l'autre peut être éventuellement substitué par un, deux ou trois groupes alkyles en $C_{1-4}$, alcoxy en $C_{1-4}$, nitro, atomes d'halogène, groupes $CF_3$ et cyano; et X est C=O, O-C=O, C=O-O, CHOH, SO, $SO_2$, O-SO, O-$SO_2$, CONH, O-CONH, O-$SO_2$NH, CO-CH=CH, C=NHOH, C=$NNH_2$; ou un groupe $R_yR_zNZ$, dans lequel $R_y$ et $R_z$ sont indépendamment un atome d'hydrogène ou un groupe alkyle en $C_{1-6}$ et Z est un groupe C=O, SO ou $SO_2$; ou un groupe $(R_wO)_2P(O)W$ dans lequel $R_w$ est un atome d'hydrogène ou un groupe alkyle en $C_{1-6}$ et W est un atome d'oxygène ou une liaison; ou

$R_1$ est un groupe cycloalkyle en $C_{3-8}$ ou un groupe alkyle en $C_{1-6}$ éventuellement substitué par un groupe qui est un groupe hydroxy, alcoxy en $C_{1-6}$, amino éventuellement substitué par un ou deux groupes alkyles en $C_{1-6}$, alcanoylamino en $C_{1-7}$, cycloalkyloxy en $C_{3-8}$ ou cycloalkylamino en $C_{3-8}$; et $R_2$ est un atome d'hydrogène; ou bien

l'un des $R_1$ et $R_2$ est un groupe nitro, cyano ou alkylcarbonyle en $C_{1-3}$ et l'autre est un groupe différent choisi parmi un groupe nitro, cyano, un atome d'halogène, un groupe alkylcarbonyle en $C_{1-3}$, méthoxy ou amino éventuellement substitué par un ou deux groupes alkyles en $C_{1-6}$ ou par un groupe alcanoyle en $C_{2-7}$; ou

$R_1$ et $R_2$ forment ensemble avec les atomes de carbone auxquels ils sont attachés un groupe 2,1,3-oxadiazole;

l'un ou l'autre des $R_3$ et $R_4$ est un atome d'hydrogène ou un groupe alkyle en $C_{1-4}$ et l'autre est un groupe alkyle en $C_{1-4}$; ou $R_3$ et $R_4$ forment ensemble un radical polyméthylène en $C_{2-5}$;

$R_5$ est un atome d'hydrogène, un groupe hydroxy, amino, alcoxy en $C_{1-6}$ ou acyloxy en $C_{1-7}$; et

E est un atome d'hydrogène; ou

$R_5$ et E forment ensemble une liaison;

soit

$R_6$ est un atome d'hydrogène ou un groupe alkyle en $C_{1-6}$; et

$R_7$ est un atome d'hydrogène, un groupe cycloalkyle en $C_{3-8}$, alcényle en $C_{2-6}$, alkyle en $C_{1-6}$ éventuellement substitué par un groupe hydroxy, alcoxy en $C_{1-6}$, alcoxycarbonyle en $C_{1-6}$, carboxy, un atome d'halogène ou un groupe amino éventuellement substitué par un ou plusieurs groupes alkyles en $C_{1-6}$; aryle ou hétéroaryle l'un ou l'autre étant éventuellement substitué par un ou plusieurs groupes choisis parmi les groupes alkyles en $C_{1-6}$, alcoxy en $C_{1-6}$, hydroxy, atomes d'halogène, groupes trifluorométhyle, nitro, cyano, acyle carboxylique en $C_{1-12}$ ou bien $R_7$ est un groupe amino ou aminocarbonyle éventuellement substitué par un ou deux groupes alkyles en $C_{1-6}$ ou substitué par un groupe cycloalkyle en $C_{3-8}$, ou un groupe $OR_9$ dans lequel $R_9$ est un groupe alkyle en $C_{1-6}$ ou benzyle éventuellement substitué de la façon définie pour $R_7$;

soit

$R_6$ et $R_7$ sont réunis pour former un radical polyméthylène en $C_{3-4}$ éventuellement substitué par un ou deux groupes alkyles en $C_{1-6}$ et contenant éventuellement un ou (lorsqu'il s'agit d'un radical polyméthylène en $C_4$) deux doubles liaisons C=C; et

X est un atome d'oxygène ou de soufre.

**2.** Composé suivant la revendication 1, dans lequel Y est un radical C-$R_1$ et a et b forment ensemble une liaison -O-.

**3.** Composé suivant les revendications 1 ou 2, dans lequel $R_1$ est un groupe nitro, cyano, acétyle, $CF_3$, $C_2F_5$ ou alkyle en $C_{1-4}$ et $R_2$ est un atome d'hydrogène.

**4.** Composé suivant la revendication 3, dans lequel $R_1$ est un groupe cyano.

**5.** Composé suivant l'une quelconque des revendications 1 à 4, dans lequel $R_3$ et $R_4$ sont chacun un groupe méthyle.

**6.** Composé suivant l'une quelconque des revendications 1 à 5, dans lequel $R_5$ est un groupe hydroxy et E est un atome d'hydrogène et la partie $R_6NCXR_7$ est trans par rapport à $R_5$.

**7.** Composé suivant l'une quelconque des revendications 1 à 6, dans lequel $R_6$ est un atome d'hydrogène et $R_7$ est un groupe phényle éventuellement substitué, un groupe alkyle en $C_{1-6}$ ramifié, alcoxy en $C_{1-6}$, benzyloxy ou amino éventuellement substitué.

**8.** Composé suivant la revendication 7, dans lequel $R_7$ est un groupe t-butylamino.

**9.** Composé choisi dans le groupe consistant en :

trans-6-cyano-2,2-diméthyl-3,4-dihydro-3-(t-butoxy-carbonyl)-amino-2H-1-benzopyran-4-ol,
trans-6-cyano-2,2-diméthyl-3,4-dihydro-3-(2-oxo-1-pipéridinyl)-2H-1-benzopyran-4-ol,
trans-6-cyano-2,2-diméthyl-3,4-dihydro-3-(4-fluorobenzoylamino)-2H-1-benzopyran-4-ol,
6-cyano-2,2-diméthyl-3,4-dihydro-3-(t-butoxy-carbonyl)-amino-2H-1-benzopyrane,
trans-6-cyano-2,2-diméthyl-3,4-dihydro-3-(t-butyl-uréido)-2H-1-benzopyran-4-ol,
trans-6-cyano-2,2-diméthyl-3,4-dihydro-3-(3,4-difluorobenzoylamino)-2H-1-benzopyran-4-ol,
trans-6-cyano-2,2-diméthyl-3,4-dihydro-3-(méthyl-uréido)-2H-1-benzopyran-4-ol,
trans-6-cyano-2,2-diméthyl-3,4-dihydro-3-acétylamino-2H-1-benzopyran-4-ol,
trans-6-cyano-2,2-diméthyl-3,4-dihydro-3-(2-pyridine-carbonylamino)-2H-1-benzopyran-4-ol,
trans-6-cyano-2,2-diméthyl-3,4-dihydro-3-(t-butyl-acétylamino)-2H-1-benzopyran-4-ol,
trans-6-cyano-2,2-diméthyl-3,4-dihydro-3-(cyclohexane-carbonylamino)-2H-1-benzopyran-4-ol,
trans-6-cyano-2,2-diméthyl-3,4-dihydro-3-(cyclohexyl-uréido)-2H-1-benzopyran-4-ol,
trans-6-cyano-2,2-diméthyl-3,4-dihydro-3-benzoylamino-2H-1-benzopyran-4-ol,
trans-6-cyano-2,2-diméthyl-3,4-dihydro-3-(isopropyl-uréido)-2H-1-benzopyran-4-ol,
trans-6-cyano-2,2-diméthyl-3,4-dihydro-3-(éthyl-uréido)-2H-1-benzopyran-4-ol,
trans-6-cyano-2,2-diméthyl-3,4-dihydro-3-(n-propyl-uréido)-2H-1-benzopyran-4-ol,
trans-6-chloro-2,2-diméthyl-3,4-dihydro-3-(t-butyl-uréido)-2H-1-benzopyran-4-ol,
trans-6-cyano-2,2-diméthyl-3,4-dihydro-3-(diméthylaminocarbonylamino)-2H-1-benzopyran-4-ol,
trans-6-cyano-2,2-diméthyl-3,4-dihydro-3-(isopropyl-thiouréido)-2H-1-benzopyran-4-ol,
trans-6-trifluorométhyl-2,2-diméthyl-3,4-dihydro-3-(t-butyl-uréido)-2H-1-benzopyran-4-ol,
trans-6-cyano-2,2-diméthyl-3,4-dihydro-3-triméthylacétylamino-2H-1-benzopyran-4-ol,
trans-6-cyano-2,2-diméthyl-3,4-dihydro-3-isobutyryl-amino-2H-1-benzopyran-4-ol,
trans-6-cyano-2,2-diméthyl-3,4-dihydro-3-(benzyloxycarbonyl)-amino-2H-1-benzopyran-4-ol,
6-cyano-2,2-diméthyl-3,4-dihydro-3-(t-butyl-uréido)-2H-1-benzopyrane,
trans-6-cyano-2,2-diméthyl-3,4-dihydro-3-(t-butoxycarbonyl)-amino-4-méthoxy-2H-1-benzopyrane et
trans-6-cyano-2,2-diméthyl-3,4-dihydro-3-(t-butoxycarbonyl)-amino-4-amino-2H-1-benzopyrane.

**10.** Procédé pour la préparation d'un composé suivant la revendication 1, qui comprend l'acylation d'un composé de formule (II) :

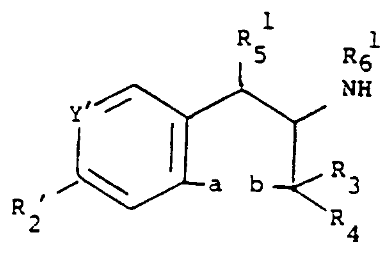

(II)

dans laquelle Y' et $R_2$' sont Y et $R_2$ respectivement ou des groupes ou des atomes pouvant être convertis en ceux-ci, $R_5{}^1$ est un groupe hydroxy, un groupe alcoxy en $C_{1-6}$, acyloxy en $C_{1-7}$ ou azide, $R_6{}^1$ est un atome d'hydrogène ou un groupe alkyle en $C_{1-6}$ et le groupe $R_6{}^1NH$ est trans par rapport au groupe $R_5{}^1$ (lorsque $R_5{}^1$ est autre qu'un azide), avec

(a) un agent acylant de formule (III) :

$R_7{}^1COQ$ (III)

dans laquelle Q est un groupe mobile et $R_7{}^1$ est $R_7$ tel que défini dans la revendication 1, ou un groupe pouvant être converti en celui-ci, lorsqu'il n'est pas réuni à $R_6$ ou (lorsque $R_6{}^1$ est un atome d'hydrogène), $R_7{}^1$ est un groupe $R_{10}$ qui est un radical polyméthylène en $C_{3-4}$ éventuellement substitué par un ou deux groupes alkyles en $C_{1-6}$ et contenant éventuellement une ou (lorsqu'il s'agit d'un radical polyméthylène en C4) deux doubles liaisons C = C, substitué en position terminale par un groupe mobile, L; et ensuite, lorsque $R_7{}^1$ est $R_{10}$, la cyclisation du composé résultant; ou
(b) lorsque $R_7$ est un groupe $NHR_{11}$ dans lequel $R_{11}$ est un atome d'hydrogène ou un groupe alkyle en $C_{1-6}$, avec un composé de formule (IV) :

$X = C = NR_{11}$ (IV)

dans laquelle X et $R_{11}$ sont tels que définis plus haut;
et ensuite, selon ce qui est désiré ou nécessaire, la conversion de $R_5{}^1$ en $R_5$, la conversion de Y' et/ou $R_2$' en Y et/ou $R_2$ la thiation éventuelle de X lorsqu'il est un atome d'oxygène en X qui est un atome de soufre et/ou la déshydratation d'un composé dans lequel $R_5{}^1$ est un groupe hydroxy pour former un composé de formule (I) dans laquelle $R_5$ et E forment une liaison, ou la conversion d'un composé de formule (I) dans laquelle la partie $R_6NCXR_7$ est trans par rapport à $R_5$ lorsqu'il est un groupe hydroxy, en un isomère cis correspondant; et éventuellement, la formation d'un sel de celui-ci acceptable du point de vue pharmaceutique.

**11.** Composé de formule (II), (V) ou (VI) :

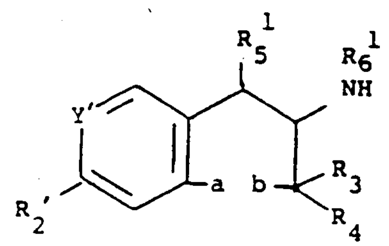

(II)

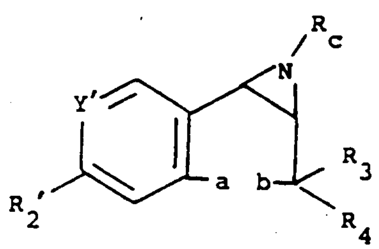

(V)

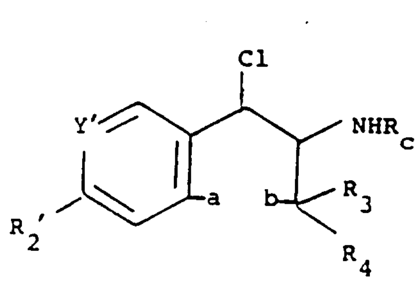

(VI)

ou $R_c$ est un groupe amino protecteur et les autres groupes variables sont telles que définies dans la

revendication 10.

12. Composé choisi dans le groupe consistant en :
trans-6-cyano-2,2-diméthyl-3,4-dihydro-3-amino-2H-1-benzopyran-4-ol,
trans-6-chloro-2,2-diméthyl-3,4-dihydro-3-amino-2H-1-benzopyran-4-ol,
trans-6-trifluorométhyl-2,2-diméthyl-3,4-dihydro-3-amino-2H-1-benzopyran-4-ol,
6-cyano-2,2-diméthyl-3,4-[(N-t-butoxycarbonyl)-aziridino]-2H-1-benzopyrane,
6-chloro-2,2-diméthyl-3,4-[(N-t-butoxycarbonyl)-aziridino]-2H-1-benzopyrane,
6-trifluorométhyl-2,2-diméthyl-3,4-[(N-t-butoxycarbonyl)-aziridino]-2H-1-benzopyrane,
6-cyano-2,2-diméthyl-3,4-[(N-benzyloxycarbonyl)-aziridino]-2H-1-benzopyrane,
trans-6-cyano-2,2-diméthyl-3,4-dihydro-3-(t-butoxycarbonyl)-amino-4-chloro-2H-1-benzopyrane,
trans-6-chloro-2,2-diméthyl-3,4-dihydro-3-(t-butoxycarbonyl)-amino-4-chloro-2H-1-benzopyrane,
trans-6-trifluorométhyl-2,2-diméthyl-3,4-dihydro-3-(t-butoxycarbonyl)-amino-4-chloro-2H-1-benzopyrane et
trans-6-cyano-2,2-diméthyl-3,4-dihydro-3-(benzyloxycarbonyl-amino)-4-chloro-2H-1-benzopyrane.

13. Composition pharmaceutique comprenant un composé suivant l'une quelconque des revendications 1 à 9 et un support acceptable du point de vue pharmaceutique.

14. Composé suivant l'une quelconque des revendications 1 à 9, utile comme substance thérapeutique active.

15. Composé suivant l'une quelconque des revendications 1 à 9, utile dans le traitement de l'hypertension et/ou des troubles des voies respiratoires.

16. Utilisation d'un composé suivant l'une quelconque des revendications 1 à 9 dans la fabrication d'un médicament utile dans le traitement de' l'hypertension et/ou des troubles des voies respiratoires.

**Revendications pour l'Etat contractant suivant : ES**

1. Procédé pour la préparation d'un composé de formule (I) ou d'un sel de celui-ci acceptable du point de vue pharmaceutique:

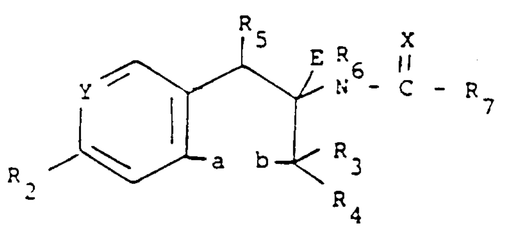

(I)

dans laquelle :

a et b forment ensemble une liaison -O- ou une liaison, ou bien un radical $CH_2$ lorsque $R_2$ est un atome d'hydrogène;

soit Y est un atome d'azote et $R_2$ est un atome d'hydrogène;

soit Y est un radical C-$R_1$

où

l'un ou l'autre des $R_1$ et $R_2$ est un atome d'hydrogène et l'autre est un groupe nitro, cyano, un atome d'halogène, un groupe $CF_3$, $C_2F_5$, formyle, aldoxime, $CF_3O$, $NO_2$-CH=CH-, NC-CH=CH-; un groupe $R_xX$- dans lequel $R_x$ est un groupe alkyle en $C_{1-6}$, aryle ou hétéroaryle, l'un ou l'autre peut être éventuellement substitué par un, deux ou trois groupes alkyles en $C_{1-4}$, alcoxy en $C_{1-4}$, nitro, atomes d'halogène, groupes $CF_3$ et cyano; et X est C=O, O-C=O, C=O-O, CHOH, SO, $SO_2$, O-SO, O-$SO_2$, CONH, O-CONH, O-$SO_2$NH, CO-CH=CH, C=NHOH, C=$NNH_2$; ou un groupe $R_yR_zNZ$, dans lequel $R_y$ et $R_z$ sont indépendamment un atome d'hydrogène ou un groupe alkyle en $C_{1-6}$ et Z est un groupe C=O, SO ou $SO_2$; ou un groupe $(R_wO)_2P(O)W$ dans lequel $R_w$ est un atome d'hydrogène ou un groupe alkyle en $C_{1-6}$ et W est un atome d'oxygène ou une liaison; ou

$R_1$ est un groupe cycloalkyle en $C_{3-8}$ ou un groupe alkyle en $C_{1-6}$ éventuellement substitué par

un groupe qui est un groupe hydroxy, alcoxy en $C_{1-6}$, amino éventuellement substitué par un ou deux groupes alkyles en $C_{1-6}$, alcanoylamino en $C_{1-7}$, cycloalkyloxy en $C_{3-8}$ ou cycloalkylamino en $C_{3-8}$; et $R_2$ est un atome d'hydrogène; ou bien

l'un des $R_1$ et $R_2$ est un groupe nitro, cyano ou alkylcarbonyle en $C_{1-3}$ et l'autre est un groupe différent choisi parmi un groupe nitro, cyano, un atome d'halogène, un groupe alkylcarbonyle en $C_{1-3}$, méthoxy ou amino éventuellement substitué par un ou deux groupes alkyles en $C_{1-6}$ ou par un groupe alcanoyle en $C_{2-7}$; ou

$R_1$ et $R_2$ forment ensemble avec les atomes de carbone auxquels ils sont attachés un groupe 2,1,3-oxadiazole;

l'un ou l'autre des $R_3$ et $R_4$ est un atome d'hydrogène ou un groupe alkyle en $C_{1-4}$ et l'autre est un groupe alkyle en $C_{1-4}$; ou $R_3$ et $R_4$ forment ensemble un radical polyméthylène en $C_{2-5}$;

$R_5$ est un atome d'hydrogène, un groupe hydroxy, amino, alcoxy en $C_{1-6}$ ou acyloxy en $C_{1-7}$; et

E est un atome d'hydrogène; ou

$R_5$ et E forment ensemble une liaison;

soit

$R_6$ est un atome d'hydrogène ou un groupe alkyle en $C_{1-6}$; et

$R_7$ est un atome d'hydrogène, un groupe cycloalkyle en $C_{3-8}$, alcényle en $C_{2-6}$, alkyle en $C_{1-6}$ éventuellement substitué par un groupe hydroxy, alcoxy en $C_{1-6}$, alcoxycarbonyle en $C_{1-6}$ carboxy, un atome d'halogène ou un groupe amino éventuellement substitué par un ou plusieurs groupes alkyles en $C_{1-6}$; aryle ou hétéroaryle l'un ou l'autre étant éventuellement substitué par un ou plusieurs groupes choisis parmi les groupes alkyles en $C_{1-6}$, alcoxy en $C_{1-6}$, hydroxy, atomes d'halogène, groupes trifluorométhyle, nitro, cyano, acyle carboxylique en $C_{1-12}$ ou bien $R_7$ est un groupe amino ou aminocarbonyle éventuellement substitué par un ou deux groupes alkyles en $C_{1-6}$ ou substitué par un groupe cycloalkyle en $C_{3-8}$, ou un groupe $OR_9$ dans lequel $R_9$ est un groupe alkyle en $C_{1-6}$ ou benzyle éventuellement substitué de la façon définie pour $R_7$;

soit

$R_6$ et $R_7$ sont réunis pour former un radical polyméthylène en $C_{3-4}$ éventuellement substitué par un ou deux groupes alkyles en $C_{1-6}$ et contenant éventuellement un ou (lorsqu'il s'agit d'un radical polyméthylène en $C_4$) deux doubles liaisons C = C; et

X est un atome d'oxygène ou de soufre;

lequel procédé comprend l'acylation d'un composé de formule (II) :

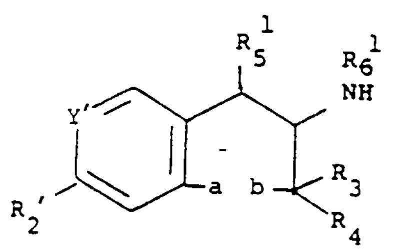

(II)

dans laquelle Y' et $R_2$' sont Y et $R_2$ respectivement ou des groupes ou des atomes pouvant être convertis en ceux-ci, $R_5{}^1$ est un groupe hydroxy, un groupe alcoxy en $C_{1-6}$, acyloxy en $C_{1-7}$ ou azide, $R_6{}^1$ est un atome d'hydrogène ou un groupe alkyle en $C_{1-6}$ et le groupe $R_6{}^1$NH est trans par rapport au groupe $R_5{}^1$ (lorsque $R_5{}^1$ est autre qu'un azide), avec

(a) un agent acylant de formule (III) :

$R_7{}^1$COQ (III)

dans laquelle Q est un groupe mobile et $R_7{}^1$ est $R_7$ tel que défini dans la revendication 1, ou un groupe pouvant être converti en celui-ci, lorsqu'il n'est pas réuni à $R_6$ ou (lorsque $R_6{}^1$ est un atome d'hydrogène), $R_7{}^1$ est un groupe $R_{10}$ qui est un radical polyméthylène en $C_{3-4}$ éventuellement substitué par un ou deux groupes alkyles en $C_{1-6}$ et contenant éventuellement une ou (lorsqu'il s'agit d'un radical polyméthylène en C4) deux doubles liaisons C = C, substitué en position terminale par un groupe mobile, L; et ensuite, lorsque $R_7{}^1$ est $R_{10}$, la cyclisation du composé résultant; ou

(b) lorsque $R_7$ est un groupe $NHR_{11}$ dans lequel $R_{11}$ est un atome d'hydrogène ou un groupe alkyle en $C_{1-6}$, avec un composé de formule (IV) :

$X=C=NR_{11}$ (IV)

dans laquelle X et $R_{11}$ sont tels que définis plus haut;
et ensuite, selon ce qui est désiré ou nécessaire, la conversion de $R_5{}^1$ en $R_5$, la conversion de Y' et/ou $R_2$' en Y et/ou $R_2$, la thiation éventuelle de X lorsqu'il est un atome d'oxygène en X qui est un atome de soufre et/ou la déshydratation d'un composé dans lequel $R_5{}^1$ est un groupe hydroxy pour former un composé de formule (I) dans laquelle $R_5$ et E forment une liaison, ou la conversion d'un composé de formule (I) dans laquelle la partie $R_6NCXR_7$ est trans par rapport à $R_5$ lorsqu'il est un groupe hydroxy, en un isomère cis correspondant; et éventuellement, la formation d'un sel de celui-ci acceptable du point de vue pharmaceutique.

**2.** Procédé suivant la revendication 1, dans lequel Y est un radical C-$R_1$ et a et b forment ensemble une liaison -O-.

**3.** Procédé suivant les revendications 1 ou 2, dans lequel $R_1$ est un groupe nitro, cyano, acétyle, $CF_3$, $C_2F_5$ ou alkyle en $C_{1-4}$ et $R_2$ est un atome d'hydrogène.

**4.** Procédé suivant la revendication 3, dans lequel $R_1$ est un groupe cyano.

**5.** Procédé suivant l'une quelconque des revendications 1 à 4, dans lequel $R_3$ et $R_4$ sont chacun un groupe méthyle.

**6.** Procédé suivant l'une quelconque des revendications 1 à 5, dans lequel $R_5$ est un groupe hydroxy et E est un atome d'hydrogène et la partie $R_6NCXR_7$ est trans par rapport à $R_5$.

**7.** Procédé suivant l'une quelconque des revendications 1 à 6, dans lequel $R_6$ est un atome d'hydrogène et $R_7$ est un groupe phényle éventuellement substitué, un groupe alkyle en $C_{1-6}$ ramifié, alcoxy en $C_{1-6}$, benzyloxy ou amino éventuellement substitué.

**8.** Procédé suivant la revendication 7, dans lequel $R_7$ est un groupe t-butylamino.

**9.** Procédé suivant la revendication 1 pour la préparation d'un composé choisi dans le groupe consistant en :

trans-6-cyano-2,2-diméthyl-3,4-dihydro-3-(t-butoxy-carbonyl)-amino-2H-1-benzopyran-4-ol,
trans-6-cyano-2,2-diméthyl-3,4-dihydro-3-(2-oxo-1-pipéridinyl)-2H-1-benzopyran-4-ol,
trans-6-cyano-2,2-diméthyl-3,4-dihydro-3-(4-fluorobenzoylamino)-2H-1-benzopyran-4-ol,
6-cyano-2,2-diméthyl-3,4-dihydro-3-(t-butoxy-carbonyl)-amino-2H-1-benzopyrane,
trans-6-cyano-2,2-diméthyl-3,4-dihydro-3-(t-butyl-uréido)-2H-1-benzopyran-4-ol,
trans-6-cyano-2,2-diméthyl-3,4-dihydro-3-(3,4-difluoro-benzoylamino)-2H-1-benzopyran-4-ol,
trans-6-cyano-2,2-diméthyl-3,4-dihydro-3-(méthyl-uréido)-2H-1-benzopyran-4-ol,
trans-6-cyano-2,2-diméthyl-3,4-dihydro-3-acétylamino-2H-1-benzopyran-4-ol,
trans-6-cyano-2,2-diméthyl-3,4-dihydro-3-(2-pyridine-carbonylamino)-2H-1-benzopyran-4-ol,
trans-6-cyano-2,2-diméthyl-3,4-dihydro-3-(t-butyl-acétylamino)-2H-1-benzopyran-4-ol,
trans-6-cyano-2,2-diméthyl-3,4-dihydro-3-(cyclohexane-carbonylamino)-2H-1-benzopyran-4-ol,
trans-6-cyano-2,2-diméthyl-3,4-dihydro-3-(cyclohexyl-uréido)-2H-1-benzopyran-4-ol,
trans-6-cyano-2,2-diméthyl-3,4-dihydro-3-benzoylamino-2H-1-benzopyran-4-ol,
trans-6-cyano-2,2-diméthyl-3,4-dihydro-3-(isopropyl-uréido)-2H-1-benzopyran-4-ol,
trans-6-cyano-2,2-diméthyl-3,4-dihydro-3-(éthyl-uréido)-2H-1-benzopyran-4-ol,
trans-6-cyano-2,2-diméthyl-3,4-dihydro-3-(n-propyl-uréido)-2H-1-benzopyran-4-ol,
trans-6-chloro-2,2-diméthyl-3,4-dihydro-3-(t-butyl-uréido)-2H-1-benzopyran-4-ol,
trans-6-cyano-2,2-diméthyl-3,4-dihydro-3-(diméthyl-aminocarbonylamino)-2H-1-benzopyran-4-ol,
trans-6-cyano-2,2-diméthyl-3,4-dihydro-3-(isopropyl-thiouréido)-2H-1-benzopyran-4-ol,
trans-6-trifluorométhyl-2,2-diméthyl-3,4-dihydro-3-(t-butyl-uréido)-2H-1-benzopyran-4-ol,
trans-6-cyano-2,2-diméthyl-3,4-dihydro-3-triméthylacétylamino-2H-1-benzopyran-4-ol,
trans-6-cyano-2,2-diméthyl-3,4-dihydro-3-isobutyryl-amino-2H-1-benzopyran-4-ol,
trans-6-cyano-2,2-diméthyl-3,4-dihydro-3-(benzyloxycarbonyl)-amino-2H-1-benzopyran-4-ol,
6-cyano-2,2-diméthyl-3,4-dihydro-3-(t-butyl-uréido)-2H-1-benzopyrane,

trans-6-cyano-2,2-diméthyl-3,4-dihydro-3-(t-butoxycarbonyl)-amino-4-méthoxy-2H-1-benzopyrane et
trans-6-cyano-2,2-diméthyl-3,4-dihydro-3-(t-butoxycarbonyl)-amino-4-amino-2H-1-benzopyrane.

**10.** Utilisation d'un composé de formule (I) suivant l'une quelconque des revendications 1 à 9 ou d'un sel de celui-ci acceptable du point de vue pharmaceutique, dans la fabrication d'un médicament utile dans le traitement de l'hypertension et/ou des troubles des voies respiratoires.